# EUROPEAN PATENT SPECIFICATION

(11) **EP 2 088 195 B1**
(45) Date of publication and mention of the grant of the patent: **13.10.2010**
(21) Application number: 08167449.1
(22) Date of filing: 23.10.2008
(51) Int. Cl.: C12N 15/09

(54) **Recombinant vector for deleting specific regions of chromosome and method for deleting specific chromosomal regions of chromosome in the microorganism using the same**
Rekombinanter Vektor zum Löschen spezifischer Chromosomenregionen und Verfahren zum Löschen spezifischer Chromosomenregionen eines Mikroorganismuschromosoms damit
Vecteur recombinant pour la suppression de régions spécifiques de chromosomes et procédé pour la suppression de régions spécifiques de chromosomes l'utilisant

(30) Priority: 11.02.2008 KR 20080012377
(43) Date of publication of application: 12.08.2009
(73) Proprietor: Korea Advanced Institute of Science and Technology, Daejeon 305-701 (KR)
(72) Inventor: Kim, Sun Chang, 305-701, Daejeon (KR); Kang, Kui Hyeon, 305-701, Daejeon (KR); Yu, Byung Jo, 305-701, Daejeon (KR); Lee, Jun Hyoung, 305-701, Daejeon (KR); Sung, Bong Hyun, 305-701, Daejeon (KR); Lee, Choong Hoon, 305-701, Daejeon (KR); Lee, Sang Hee, 305-701, Daejeon (KR); Lee, Ju Young, 305-701, Daejeon (KR); Park, Myung Keun, 305-701, Daejeon (KR)
(74) Representative: Grosse, Wolfgang

(56) References cited:
- MURPHY ET AL: "Use of bacteriophage lambda recombination funtions to promote gene replacement in Escherichia coli" JOURNAL OF BACTERIOLOGY, AMERICAN SOCIETY FOR MICROBIOLOGY, US, vol. 180, no. 8, 1 April 1998 (1998-04-01), pages 2063-2071, XP002149589 ISSN: 0021-9193
- DATSENKO KIRILL A ET AL: "One-step inactivation of chromosomal genes in Escherichia coli K-12 using PCR products" PROCEEDINGS OF THE NATIONAL ACADEMY OF SCIENCES OF USA, NATIONAL ACADEMY OF SCIENCE, WASHINGTON, DC.; US, vol. 97, no. 12, 6 June 2000 (2000-06-06), pages 6640-6645, XP002210218 ISSN: 0027-8424

## Description

### BACKGROUND OF THE INVENTION

### Field of the Invention

The present invention relates to a recombinant vector for deletion of specific chromosomal regions, which is capable of providing efficient and easy simultaneous deletion of specific chromosomal regions of a target microbe and a method for deletion of targeted microbial chromosomal regions using the same.

### Description of the Related Art

Striking development of biotechnologies opened the era of post-genomics. Keeping pace with current trends, industrial strains that can be widely and beneficially used in biotechnology industry have been produced taking advantage of genetic information of diverse organisms. However, a large majority of currently available industrial strains have problems such as excessive energy consumption and by-product production by gene clusters producing useless materials and therefore are not favorable for industrial-scale production of high-purity useful materials. To this end, a great deal of research has been focused on development of techniques which are capable of accomplishing rapid, efficient and convenient deletion of industrially useless gene clusters from genomes of target microbes, for artificial construction of novel high-functionality metabolically engineered strains and cell lines with remarkably enhanced productivity through the deletion of useless gene clusters from the selected microbial genomes.

Conventional known techniques for deletion of specific regions of *Escherichia coli* (*E. coli*) chromosomes involve use of a linear DNA fragment containing a recombination region of a specific region to be deleted and a selectable marker, in conjunction with two different vectors having functions necessary for genomic insertion and deletion of the linear DNA fragment. Specifically, in a first step of the conventional gene deletion strategy, a targeted chromosomal region is deleted through recombinational insertion of a linear DNA fragment having a selectable marker into a specific chromosomal region to be deleted, using a vector having recombination functions. This is followed by removal of the introduced vector from *E. coli.* In a second step, a vector intended for removal of the selectable marker of the linear DNA fragment which was used in deletion of the specific chromosomal region is introduced into the *E. coli* host to thereby remove the selectable marker gene. Therefore, when it is desired to delete multiple specific chromosomal regions of *E. coli* using the conventional gene deletion methodology, it is disadvantageous in that the conventional technique requires sequential introduction and subsequent removal of two vectors necessary for each deletion step, thus resulting in complicated and time-consuming processes.

### SUMMARY OF THE INVENTION

Therefore, the present invention has been made in view of the above problems, and it is an object of the present invention to provide a recombinant vector for deletion of specific gene regions, which is capable of achieving rapid, efficient and successive deletion of specific gene regions of a microbe, and a method for deletion of specific microbial chromosomal regions using the same.

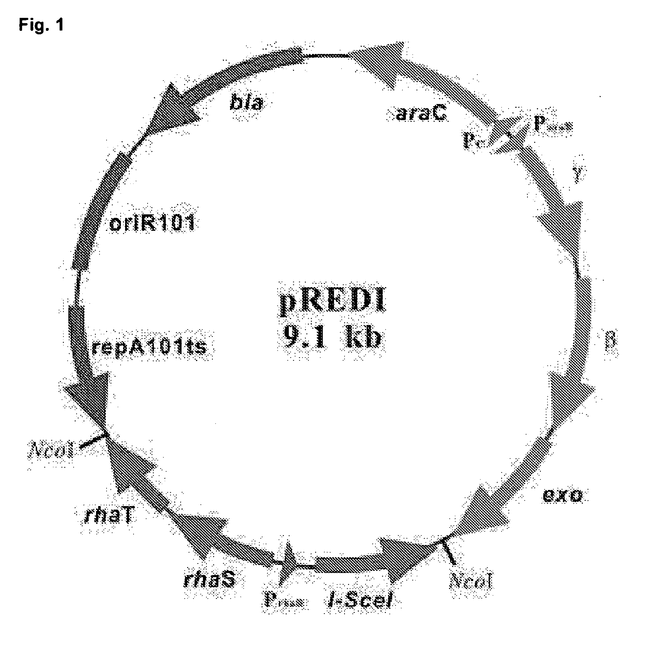

In accordance with an aspect of the present invention, the above and other objects can be accomplished by the provision of a recombinant vector for deletion of specific chromosomal regions, comprising an arabinose-inducible promoter (Pₐᵣₐ); a gene encoding a protein involved in lambda (λ)-red recombination; a rhamnose-inducible promoter (Pₘₐ); and a gene encoding the I-*Sce*I endonuclease, wherein the vector has a base sequence of SEQ ID NO: 1 and is represented by a cleavage map of FIG. 1.

In accordance with another aspect of the present invention, there is provided *Escherichia coli* transformed with the aforesaid recombination vector.

In accordance with yet another aspect of the present invention, there is provided a method for deletion of specific chromosomal regions of a microbe using the aforesaid recombination vector, comprising the steps of:
1) preparing a linear DNA fragment containing homology arms A and B which are involved in λ-red recombination when they are introduced into a target microbe; a selectable marker; an I-*Sce*I recognition site which is involved in homologous recombination for removal of the selectable marker; and a homology arm C which is involved in homologous recombination for removal of the selectable marker;
2) introducing the linear DNA fragment into a microbe transformed with the aforesaid recombination vector to replace a specific locus of the microbial chromosome with the linear DNA fragment through λ-red recombination between the homology arms of the DNA fragment and the microbial chromosome regions homologous to the homology arms; and
3) culturing the specific chromosomal locus-replaced microbe in a rhamnose-containing medium to induce expression of the I-*Sce*I endonuclease, such that homologous recombination between the homology arm C of the DNA fragment and the microbial chromosomal region homologous to the homology arm C is driven to remove the selectable marker,
   wherein the homology arm A is a region homologous to 50 to 500bp of one end of the deletion target domain of a microbial chromosome and the homology arm B is a region homologous to 50 to 500bp of the other end of the deletion target domain of the microbial chromosome, and
   the homology arm C is a region that is homologous to a 300-500bp region contiguous to either one of the microbial chromosome regions homologous to the homology arm A and homology arm B.

In one embodiment of the present invention, the method may include repeating Steps 1 and 2 to prepare a plurality of different linear DNA fragments and introducing the linear DNA fragments into a microbe transformed with a recombination vector of claim 1 to delete a plurality of specific microbial chromosomal regions.

In one embodiment of the present invention, the selectable marker may be at least one selected from the group consisting of a chloramphenicol-resistant gene having a base sequence of SEQ ID NO: 13, a kanamycin-resistant gene having a base sequence of SEQ ID NO: 14, and sacB.

In one embodiment of the present invention, the method may further comprise culturing the microbes in a sucrose-containing medium after the step of removing the selectable marker.

In one embodiment of the present invention, the homology arm A, the homology arm B and the homology arm C may have base sequences of SEQ ID NO: 15, SEQ ID NO: 16 and SEQ ID NO: 17, respectively. In another embodiment of the present invention, the homology arm A, the homology arm B and the homology arm C may have base sequences of SEQ ID NO: 22, SEQ ID NO: 23 and SEQ ID NO: 24, respectively. In yet another embodiment of the present invention, the homology arm A, the homology arm B and homology arm C may have base sequences of SEQ ID NO: 31, SEQ ID NO: 32 and SEQ ID NO: 33, respectively.

In one embodiment of the present invention, the specific chromosomal region of the microbe may contain a gene essential for survival of the microbe, and the linear DNA fragment may further contain the aforesaid essential survival gene between the homology arm A and the homology arm C.

In one embodiment of the present invention, the essential gene is *arg*S having a base sequence of SEQ ID NO: 43, and the homology arm A, the homology arm B and the homology arm C may have base sequences of SEQ ID NO: 40, SEQ ID NO: 41 and SEQ ID NO: 42, respectively.

### BRIEF DESCRIPTION OF THE DRAWINGS

FIG. 1 is a cleavage map of a recombinant vector pREDI for deletion of chromosomes in accordance with the present invention;
FIG. 2 is a schematic diagram illustrating deletion of a selectable marker, using a recombination vector pREDI in accordance with the present invention;
FIG. 3a is a diagram showing insertion of two linear DNA fragments containing different selectable markers into two specific genomic regions of *E. coli* and simultaneous deletion of the inserted linear DNA fragments, using a recombination vector pREDI in accordance with the present invention;

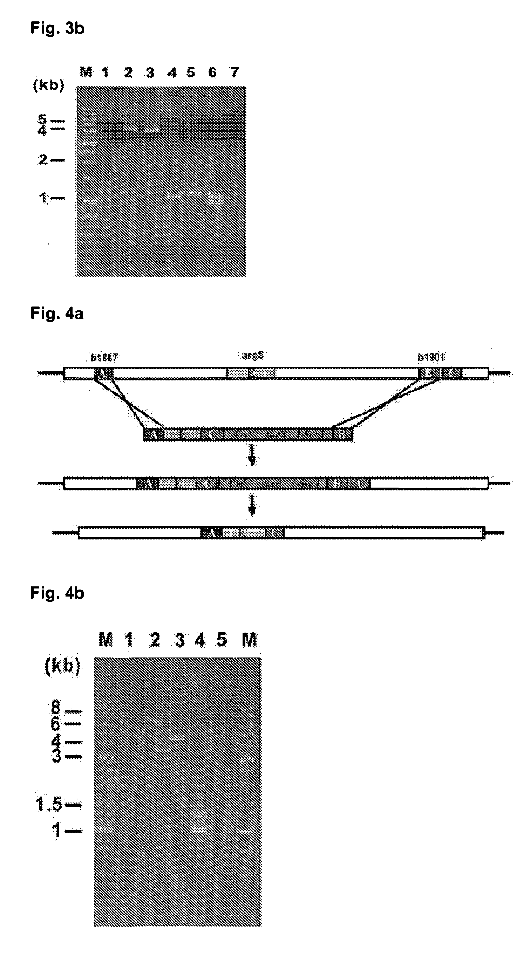

FIG. 3b is a photograph showing electrophoretic patterns for the results of PCR amplification conducted to confirm the insertion of two linear DNA fragments and the degree of genomic deletion after insertion and deletion of two linear DNA fragments into and from two specific genomic regions of *E*. *coli* (M: marker, Lane 1: MG1655 strain not transformed with a linear DNA fragment (positive control), Lane 2: PCR results confirming that a DNA fragment A-C-Cm *sac*B I-S*ec*I-B, constructed to delete a b0980-b1052 region, was successfully introduced into the *E. coli* strain, Lane 3: PCR results confirming that a DNA fragment A-C-Km *sac*B I-SecI-B, constructed to delete a b1137-b1168 region, was successfully introduced into the *E. coli* strain, Lane 4: PCR results confirming that a DNA fragment A-C-Cm *sac*B I-S*ec*I-B, constructed to delete a b0980-b1052 region, was successfully introduced into the *E. coli* strain, and a Cm *sac*B I-SecI-B portion was then removed by the action of I-S*ec*I, Lane 5: PCR results confirming that a DNA fragment A-C-Km *sacB* I-*Sec*I-B, constructed to delete a b1137-b1168 region, was successfully introduced into the *E. coli* strain, and a Km *sac*B I-*Sec*I-B portion was then deleted by the action of I-S*ec*I, and Lanes 6 and 7: PCR results for each region between b0980-b1052 and between b1137-b1168, conducted to confirm whether b0980-b1052 and b1137-b1168 were successfully deleted from the *E*. *coli* strain);
FIG. 4a is a diagram illustrating deletion of a specific genomic region containing an essential survival gene, in deletion of specific genomic regions using a recombination vector pREDI in accordance with the present invention; and
FIG. 4b is a photograph showing electrophoretic patterns of the results of PCR amplification conducted to confirm the insertion of a linear DNA fragment and the degree of genomic deletion, after deletion of a specific genomic region containing an essential survival gene (Lane 1: MG1655 strain not transformed with linear DNA fragment, Lane 2: MG1655 strain transformed with a linear DNA fragment, Lane 3: MG1655 strain transformed with a linear DNA fragment, Lane 4: MG1655 strain with no deletion of chromosome, and Lane 5: MG1655 strain with deletion of a specific chromosomal region).

### DETAILED DESCRIPTION OF THE PREFERRED EMBODIMENTS

As a result of a variety of extensive and intensive studies and experiments to solve the problems as described above, *i.e*. disadvantages of a conventional art requiring the use of two vectors for λ-red recombination and I-*Sce*I endonuclease expression functions which are employed in deletion of specific chromosomal regions of microorganisms, the inventors of the present invention succeeded in construction of a recombination vector pREDI which enables expression of the above-mentioned two functions by a single vector using different induction methods. Therefore, the present invention relates to a recombinant vector pREDI for deletion of specific chromosomal regions of a microbe, *E. coli* transformed with the same recombination vector pREDI, and a method for deletion of specific chromosomal regions of a microbe, using a linear DNA fragment containing the recombination vector pREDI and a selectable marker.

More specifically, the present invention provides a recombinant vector for deleting specific chromosomal regions of a microbe, comprising an arabinose-inducible promoter (Pₐᵣₐ); a gene encoding a protein involved in lambda (λ)-red recombination; a rhamnose-inducible promoter (Pᵣₕₐ); and a gene encoding the I-*Sce*I endonuclease, wherein the recombinant vector has a base sequence of SEQ ID NO: 1 and is represented by a cleavage map of FIG. 1.

In the context of the present invention, the protein involved in λ-red recombination is a conjugated protein consisting of Gam, Bet and Exo, as disclosed in the literature including Datsenko, K. A. et al., each of which is expressed by y, β and exo genes. Gam inhibits the host RecBCD exonuclease V so that Bet and Exo can gain access to DNA ends to promote recombination (Datsenko, K. A. et al., Proc. Natl. Acad. Sci. 97:6640, 2000; Murphy, K. C., J. Bacteriol., 180:2063,1998).

For expression of the protein involved in arabinose-inducible λ-red recombination in the context of the present invention, the aforesaid recombination vector contains a gene encoding the λ-red recombination protein, *e.g*. γ/β/exo gene (SEQ ID NO: 3), in conjunction with the arabinose-inducible promoter (Pₐᵣₐ) (SEQ ID NO: 2). Further, the λ-red recombination protein (γ, β, exo) allows the occurrence of λ-red recombination upon homologous recombination-mediated insertion of the linear DNA fragment into a target chromosome of interest. A gene encoding Gam, a gene encoding Bet and a gene encoding Exo may have base sequences of SEQ ID NO: 4, SEQ ID NO: 5 and SEQ ID NO: 6, respectively.

Further, the recombination vector in accordance with the present invention is configured to exert rhamnose-inducible I-SceI expression which consequently results in specific cleavage of the I-*Sce*I recognition site. According to such a configuration, the thus-expressed I-*Sce*I endonuclease cleaves the I-*Sce*I recognition site contained in the linear DNA fragment incorporated into the genome to thereby facilitate homologous recombination between the partial chromosomal fragment contained in the linear DNA and the microbial chromosomal region homologous to that chromosomal fragment. In order to achieve rhamnose-inducible I-SceI expression, the recombination vector of the present invention may comprise the rhamnose-inducible promoter (Pᵣₕₐ) (SEQ ID NO: 7) and a gene encoding the endonuclease I-*Sce*I (SEQ ID NO: 8).

Further, the present invention relates to *E. coli* transformed with the aforesaid recombination vector.

In the context of the present invention, the method of deleting specific chromosomal regions of a microbe via the use of a linear DNA fragment containing the recombination vector pREDI and the selectable marker specifically includes the following steps:
(1) preparing a linear DNA fragment containing a selectable marker, a sacB gene, an I-*Sce*I recognition site and homology arms that are partially homologous to a microbial chromosome;
(2) transforming the pREDI-transformed microbe with the aforesaid linear DNA fragment to thereby replace the target chromosomal region of the microbe with the linear DNA fragment of Step 1 by a λ-red recombination protein that is expressed under the control of an arabinose-inducible promoter in the recombination vector pREDI; and
(3) eliminating the remaining selectable marker from the microbial genome, by expression of the I-*Sce*I endonuclease that is under the control of a rhamnose-inducible promoter of the recombination vector pREDI harboring in the DNA fragment-transformed microbe.

Further, the above deletion method may further comprise Step 4 of simultaneously deleting two or more genomic regions of the microbe through insertion of two or more linear DNA fragments containing different selectable markers.

In addition, the above method may comprise an additional step 5 of deleting a specific region containing a gene essential for survival of a microbe. Therefore, the genomic deletion method of the present invention enables efficient deletion of only the desired specific genomic region within a short period of time while retaining the essential gene, using a linear DNA fragment containing the essential gene.

In Step 1 of the method of deleting a specific microbial chromosomal region using the recombinant vector of the present invention for deletion of specific chromosomal regions, the linear DNA fragment contains a selectable marker; 50-500bp homology arms A and B involved in λ-red recombination and homologous to a portion of a microbial chromosome; an I-*Sce*I recognition site necessary for the removal of the selectable marker; a 300-500bp homology arm C; and a sacB gene to confirm the markerless deletion of the target regions.

Examples of the selectable marker that can be used in the present invention may include, but are not limited to, a chloramphenicol-resistant gene (Cm^{R}), a kanamycin-resistant gene (Km^{R}), and tetracycline-resistant gene (Tc^{R}). A mutant strain harboring a chromosome which is recombined with a DNA fragment containing such a selectable marker gene acquires resistance to the corresponding antibiotic, which enables selection of the mutant strain devoid of a specific chromosomal region.

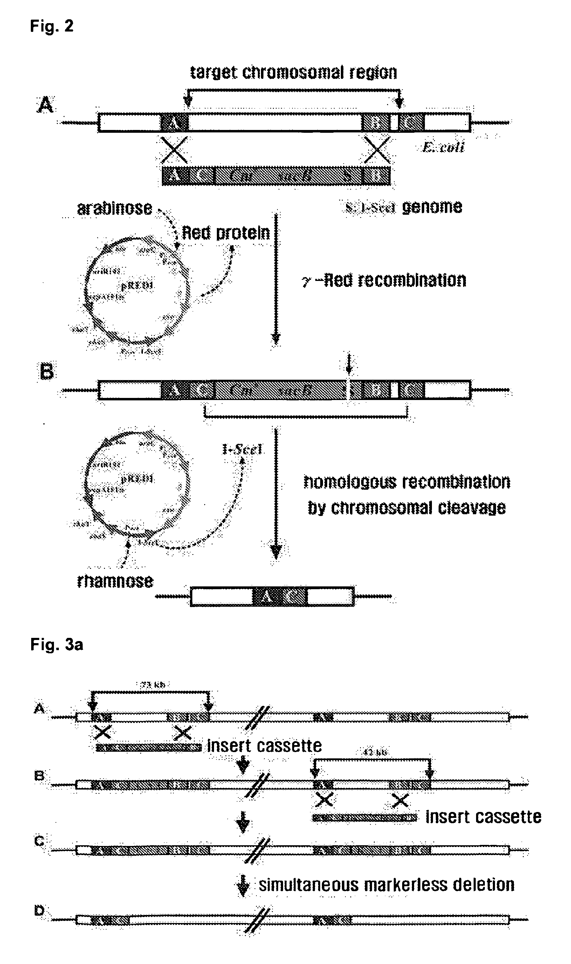

As shown in FIG. 2, the homology arm refers to a region homologous to a microbial chromosome which is sought to be modified or deleted, and is designated as homology arms A, B and C, respectively, in sequence lists and drawings. Homology arms A and B are obtained by PCR amplification of 50-500bp flanking sequences on both ends of the microbial chromosome to be deleted. They are positioned at respective opposite ends of the linear DNA fragment and participate in λ-red recombination for introduction of the linear DNA fragment into the microbe. The homology arm C is obtained by PCR amplification of a 50-500bp flanking sequence on either end of the microbial chromosome homologous to the homology arm A or B, is positioned next to a homologous region on either end of the linear DNA fragment, and is involved in the homologous recombination for removal of the selectable marker. Herein, the homology arms A, B and C may vary depending on kinds of target microorganisms and genomic regions to be deleted. In one embodiment of the present invention, each of the homology arms A, B and C may have a base sequence of SEQ ID NO: 15, 16 and 17, SEQ ID NO: 22, 23 and 24, SEQ ID NO: 31, 32 and 33, or SEQ ID NO: 40, 41 and 42, even though they are not limited thereto.

The I-*Sce*I recognition site (SEQ ID NO: 52) is 18bp in length. Since the *E. coli* chromosome contains no I-*Sce*I recognition site, the linear DNA fragment used for deletion of a specific gene must contain the I-*Sce*I recognition site.

In Step 2, a microbial strain is transformed with a recombination vector by a conventional electroporation technique, and the linear DNA fragment is then electroporated into the microbial strain. As shown in FIG. 2, a targeted chromosomal site is deleted by replacement of a specific site of the microbial strain chromosome with the DNA fragment, through λ-red recombination between the homology arms A and B located respectively on opposite ends of the linear DNA fragment and homologous to the microbial chromosome and the corresponding homologous microbial chromosome region. Then, the microbial mutants having a chromosomal replacement with the linear DNA fragment are cultured and selected in a medium containing the corresponding antibiotic depending on the kinds of selectable markers. Further, the λ-red recombination-mediated gene replacement in the thus-selected mutants can be confirmed by PCR.

In Step 3, as shown in FIG. 2, the microbial mutants replaced with the linear DNA fragment are selected and cultured to allow rhamnose-inducible I-*Sce*I expression, which, in turn, results in promotion of the homologous recombination between the homology arm C positioned at one end of the linear DNA fragment and the microbial chromosome region homologous to the homology arm C, thus leading to deletion of the selectable marker from the microbial mutants. Since transcription of an I-*Sce*I gene in the recombination vector is under the control of a rhamnose-inducible promoter, mutants harboring the recombination vector introduced therein are cultured in a medium supplemented with rhamnose to induce expression of the I-*Sce*I gene, which consequently results in site-specific cleavage of the I-*Sce*I recognition site. In this manner, specific cleavage of the I-*Sce*I recognition site facilitates homologous recombination between the region of the linear DNA fragment having homology with a microbial chromosome portion and the corresponding homologous chromosomal region of the microbe.

Further, the microbial mutants are cultured in a sucrose-containing medium to thereby express a sacB gene harbored in the linear DNA fragment introduced into the microbial mutants, thus causing toxicity to cells. Generally, the sacB gene encodes exoenzyme levansucrase which catalyzes degradation of sucrose into glucose and fructose and synthesis of levan, a polymer of fructose. Because the levan is toxic to cells, microorganisms having the sacB gene (SEQ ID NO: 53) cannot survive in a sucrose-containing medium. Therefore, the microbial mutants having homologous recombination-mediated deletion of the selectable marker can be selected in a sucrose-containing medium.

In order to delete two different genomic regions in Step 4 with modification of the above method, two linear DNA fragments containing different selectable markers, *e.g*. chloramphenicol-resistant gene (Cm^{R}) and kanamycin-resistant gene (Km^{R}), are constructed by PCR, as shown in FIG. 3a. A first linear DNA fragment is electroporated into the microbes transformed with the recombination vector of the present invention, a specific chromosomal locus of the microbial transformants is replaced with the linear DNA fragment through λ-red recombination of the recombination vector, the microbes are cultured and selected in a medium containing an antibiotic of interest corresponding to the kinds of selectable markers, and finally gene replacement on the chromosome of microbial strains is verified by PCR. Next, a second linear DNA fragment having another selectable marker is electroporated into the target strains, a specific chromosomal locus of the microbial transformants is replaced with the linear DNA through λ-red recombination, the microbes are cultured and selected in a medium containing a pertinent selectable marker, and finally gene replacement on the chromosome of the microbial strains is confirmed by PCR (FIG. 3b).

Further, it is possible to select microbial strains with deletion of two selectable markers as follows. First, the microbial strains having microbial chromosomal replacements with two linear DNA fragments are cultured in a rhamnose-containing medium to induce expression of an I-*Sce*I gene which consequently results in specific cleavage of an I-*Sce*I recognition site, thus leading to homologous recombination between the homology domains of the linear DNA fragment homologous to the microbial chromosome and the corresponding homologous microbial chromosomal region. Simultaneously with homologous recombination, a sacB gene of the linear DNA fragment introduced into the microbial mutant strains is expressed to induce cell toxicity. Therefore, the microbial mutants having homologous recombination-mediated deletion of two selectable markers can be selected in a sucrose-containing medium. In this manner, it is advantageous to greatly reduce the time necessary for deletion of targeted genomic regions through single-deletion procedure of the selectable markers recombinantly inserted into two different regions. Deletion of the selectable markers through homologous recombination can be verified by PCR (FIG. 3b). Even though the use of two linear DNA fragments was exemplified in the deletion of specific genomic regions, it is also possible to additionally delete multiple specific regions of the chromosome, using two or more linear DNA fragments.

As to Step 5 of the present invention method, deletion of a genomic region containing essential gene(s) is not feasible with a conventional genomic deletion strategy. In other words, the conventional method of deleting the essential gene-containing region disadvantageously involves division of the deletion target region into two genomic regions with respect to the essential gene and then deletion of the target gene by two deletion steps. Step (5) of the present invention method is intended to solve such a disadvantage. In order to achieve one-step deletion of a target genomic region having essential gene(s), a linear DNA fragment containing the essential gene(s) present in the target genomic region is constructed and the target genomic region is then replaced with the resulting linear DNA fragment construct using the recombination vector pREDI in accordance with the present invention.

As used herein, the term "essential gene" refers to a gene that is essentially necessary for survival of *E*. *coli,* which includes, for example, *arg*S. Specifically, examples of 300 essential genes are disclosed in Baba T, Ara T, Hasegawa M, Takai Y, Okumura Y, Baba M, Datsenko KA, Tomita M, Wanner BL, Mori H.; Construction of Escherichia coli K-12 in-frame, single-gene knockout mutants: the Keio collection.; Mol Syst Biol. 2006;2:2006.0008. Epub 2006 Feb 21, page 5, line 9 (supplement data).

For example, as shown in FIG. 4a, a linear DNA fragment is constructed to contain an essential gene (E) (such as *arg*S) between homology arms A and C and then a specific genomic region of *E. coli* is replaced with the resulting DNA fragment construct. As shown in Step 3, an *E. coli* strain with markerless deletion of a specific chromosomal region containing is selected by homologous recombination according to expression of I-*Sce*I endonuclease and additionally, cell culture of *E. coli* in a sucrose-containing medium. In this manner, through the use of an essential gene-containing linear DNA fragment for deletion of a target genomic region having essential gene(s), it is possible to select *E. coli* strains having clean deletion of useless gene clusters from the selected genomic region while retaining the existing essential gene(s). Such genomic modifications can be verified by PCR (FIG. 4b).

As described before, it is possible to delete two or more specific gene regions or otherwise to successively delete specific gene regions of a microbial chromosome additionally containing essential gene(s), by repeating the steps of constructing a linear DNA fragment having a specific homology arm only and introducing the resulting construct into the target strain while rendering the strain to retain the recombination vector.

Even though *E. coli* is used as a subject microorganism, the present invention is not limited thereto. The linear DNA replacement is conducted based on the λ-red recombination method. The λ-red recombination-mediated replacement of an *E. coli* chromosome with a linear DNA fragment obtained by PCR amplification is carried out as described by Datsenko KA et al, PNAS, 97:6640. The homologous recombination process of deleting a selectable marker, which is promoted by cleavage of an *E. coli* chromosome, is conducted as described by Posfai G, et aL, Nucleic Acids Res., 27:4409. 1999. In addition, use of the sacB gene as a selectable marker due to expression of toxicity to sucrose is disclosed in Van der Geize R, et al., FEMS Microbiol. Lett., 205:197, 2001.

Therefore, the present invention provides a rapid, efficient and markerless deletion method of a target genomic region which involves replacement of a specific locus of a microbial chromosome with a linear DNA fragment via λ-red recombination, subsequent removal of the selectable marker by I-*Sce*I-mediated chromosomal cleavage in conjunction with induction of sacB-mediated sucrose toxicity, and finally expression of the gene under the control of two different promoters in the recombination vector pREDI. This method can be adapted for the construction of minimized genomes.

### EXAMPLES

Now, the present invention will be described in more detail with reference to the following Examples. These examples are provided only for illustrating the present invention and should not be construed as limiting the scope and spirit of the present invention.

### Example 1 : Construction of recombinant vector pREDI for deletion of specific chromosomal regions

A recombination vector in accordance with the present invention was constructed by cloning a recombinant PCR gene product rhaTS-Pᵣₕₐ-I-*Sce*I fragment, which contains an *NcoI* recognition site and expresses I-*Sce*I endonuclease under the control of rhamnose-inducible promoter (Pᵣₕₐ), into the *Nco*I site of a template vector pKD46 (Datsenko KA, et al., *PNAS,* 97:6640, 2000) which contains λ-red recombination functions under the control of an arabinose-inducible promoter (Pₐᵣₐ). Specifically, the rhaTS-Pᵣₕₐ-I-*Sce*I fragment was constructed as follows. A PCR product rhaTS-P*rha* fragment was obtained by performing PCR amplification in an *E. coli* MG1655 genome using 25 pmoles of a primer NcoI-rha (SEQ ID NO: 9) and Pᵣₕₐ(SEQ ID NO: 10). A PCR product I-*Sce*I fragment was obtained by performing PCR amplification in pST76-ASceP (Posfai G, et al., Nucleic Acids Res., 27:4409, 1999) using primers I-*Sce*I-F (SEQ ID NO: 11) and *Nco*I-I-*Sce*I (SEQ ID NO: 12). Then, the resulting rhaTS-P*rha* and I-*Sce*I fragments were amplified by recombinant PCR, using primers *Nco*I-rha and *Nco*I-I-*Sce*I*.* The resulting PCR product was digested with *Nco*I and cloned into the *Nco*I site of the plasmid pKD46 to thereby construct a recombination vector pREDI in accordance with the present invention. A cleavage map of the recombination vector pREDI is shown in FIG. 1. Base sequences of PCR primers are as follows:
**Primer *Nco*I-rha** (SEQ ID NO: 9):
   5'-CATGCCATGGGGCATGGCGAATTAATCTTTCTG-3'
**Primer Pᵣₕₐ** (SEQ ID NO: 10):
   5'-ATGTGATCCTGCTGAATTTCATTACGACCAGTCT-3'
**Primer I-*Sce*I-F** (SEQ ID NO: 11):
   5'-TTAGACTGGTCGTAATGAAATTCAGCAGGATCACATAATGCATCAAAAA AAC CAGGTAATGAACCTGGG-3'
**Primer *Nco*I-I*-Sce*I** (SEQ ID NO: 12):

### Example 2: Deletion of specific chromosomal regions of microbes using pREDI vector

### (1) Construction of linear DNA fragments

From *E. coli* strain K-12 MG1655 (by courtesy of Dr. Jung-Hye Roe, Department of Microbiology, Seoul National University, Seoul, Korea), *E. coli* mutants where a useless gene cluster was deleted from the genome were obtained according to the following procedure.

First, the Cm^{R} gene (SEQ ID NO: 13) of a pSG76 vector (Posfai G, et al., Nucleic Acid Res., 27:4409, 1999) was digested with two restriction endonucleases *Kpn*I and *Bam*HI (New England Biolabs, Beverly, MA) and cloned into the *Kpn*I and *Bam*HI sites of a pST76K vector (Posfai G, et al., Nucleic Acid Res., 27:4409, 1999) containing the I-*Sce*I recognition site. Then, the sacB gene from a pDELTA vector (GibcoBRL-DELETION FACTORY SYSTEM VERSION 2.) was cleaved with *Bam*HI (New England Biolabs, Beverly, MA) and ligated into the *Bam*HI site of the above plasmid construct with the Cm^{R} gene and I-*Sce*I recognition site, using a ligase (New England Biolabs, Beverly, MA). The constructed plasmid was designated as pSCI.

Further, the *Bam*HI-cut sacB gene was ligated into the *Bam*HI site of a pST76-K vector (Posfai G, et al., Nucleic Acid Res., 27:4409, 1999) containing the Km^{R} gene (SEQ ID NO: 14) and the I-*Sce*I recognition site, using ligase (New England Biolabs, Beverly, MA). The resulting vector was designated as pSKI.

As shown in FIG. 2, linear DNA fragments (ca. 3.5kb) containing three homology arms A (SEQ ID NO: 15), B (SEQ ID NO: 16) and C (SEQ ID NO: 17), and chloramphenicol-resistant gene (Cm^{R}, SEQ ID NO: 13), sacB (SEQ ID NO: 53) and I-*Sce*I cleavage site (S, SEQ ill NO: 52) as selectable markers were constructed by recombinant PCR with an *E. coli* MG1655 chromosome and the above-constructed plasmid pSCI or pSKI as a template. One short (50bp) terminal fragment (A: SEQ ID NO: 15) and one homology arm (500bp) (C: SEQ ID NO: 17) were PCR-amplified using the *E*. *coli* K-12 MG1655 chromosome as a template and primers b0004A-F (SEQ ID NO: 18) and b0025C-R (SEQ ID NO: 19). The Cm^{R}-sacB-S fragment (CSI fragment) and the other short terminal fragment (B: SEQ ID NO: 16) were PCR-amplified using the plasmid pSCI as a template and primers CSI-f (SEQ ID NO: 20) and b0024B-R (SEQ ID NO: 21). Subsequently, the intact linear DNA fragments A-C-Cm^{R}-sacB-S-B were prepared by recombinant PCR, using A-C fragments and the PCR products Cm^{R}-sacB-S-B and Km^{R}-sacB-S-B as templates and primers b0004A-F (SEQ ID NO: 18) and b0024B-R (SEQ ID NO: 21). The thus-amplified DNAs were isolated using Nucleogen Gel-Extraction KIT (NucleoGen Inc., Gyeonggi-do, Korea). The linear DNA fragment obtained as above had a base sequence of SEQ ID NO: 54.

Base sequences of the PCR primers are as follows:
**Primer b0004A-F** (SEQ ID NO: 18):
**Primer b0025C-R** (SEQ ID NO: 19):
**Primer CSI-F** (SEQ ID NO: 20):
   5'-TACGACTCACTATAGGGAGACCGGAATTCG-3'
**Primer b0024B-R** (SEQ ID NO: 21):

### (2) Deletion of specific genomic regions of microbes by introduction of linear DNA fragments into E. coli strain transformed with pREDI vector

The above-constructed linear DNA fragment was transferred into an *E*. *coli* strain MG1655 harboring the pREDI vector by a standard electroporation method (Bio-RAD, Bacterial electro-transformation and Plus Controller Instruction Manual, Cat. NO 165-2098; Thompson, JR, et al. Yeast 14:565, 1998; and Grant, SG, et al. Proc. Natl. Acas. Sci. USA, 87:4645, 1990). Since the linear DNA fragment contains two homology arms A and B that are homologous to the chromosomal region of the above *E. coli* strain, the genomic region (b0004-b0024) flanked by these two homology arms was deleted and then replaced with the linear DNA fragment The *E. coli* mutants containing a gene replacement with the linear DNA fragment exhibit chloramphenicol-resistance due to the presence of a Cm^{R} gene in the linear DNA fragment, so they were selected in a chloramphenicol-containing medium.

Since the transcription of an I-*Sce*I gene, which is present on the pREDI vector with selectable markers in the *E. coli* mutants having a gene cluster replacement of the *E. coli* b0004-b0024 region, is under the control of a rhamnose-inducible promoter, the *E*. *coli* mutants were cultured in a rhamnose-containing medium to thereby induce expression of the I*-Sce*I endonuclease. By additional incorporation of sucrose into the culture medium during this process, bacterial selection was induced using a sacB gene present on the linear DNA fragment introduced into *E. coli.* That is, I-*Sce*I-mediated restriction of the target microbial chromosomal region resulted in homologous recombination between the microorganism-derived region present on the introduced linear DNA fragment and the corresponding homologous region of the microbial chromosome, which leads to deletion of the selectable marker and sacB gene region from the *E. coli* chromosome, consequently rendering *E. coli* strains to lose levan toxicity. Therefore, the mutant strains with deletion of the selectable marker can be selected in a sucrose-containing medium (see FIG. 2). Deletion of the b0004-b0024 gene from the thus-selected mutant strains was confirmed by PCR. The mutant strain was designated as Δ(b0004-b0024).

### Example 3: Deletion of two specific genomic regions using insertion and deletion of linear DNA fragments containing two different selectable markers into/from specific genomic region

Two linear DNA fragments containing two different selectable markers were constructed. Analogously to Example 2, the constructed DNA fragments were sequentially replaced into the chromosome, followed by simultaneous deletion of two different genomic regions.

First, b0980-b1052 and b1137-b1168 regions of an *E. coli* genome were selected as two genomic regions to be deleted. For deletion of the b0980-b1052 region, a linear DNA fragment containing a chloramphenicol-resistant gene (Cm^{R}) was constructed analogously to Example 1, using homology arm A having a base sequence of SEQ ID NO: 22, homology arm B having a base sequence of SEQ ID NO: 23 and homology arm C having a base sequence of SEQ ID NO: 24, and primers b0980A-F (SEQ ID NO: 25), b0980A-R (SEQ ID NO: 26), b1051B-F (SEQ ID NO: 27), b1051B-R (SEQ ID NO: 28), b1052C-F (SEQ ID NO: 29) and b1052C-R (SEQ ID NO: 30). The resulting linear DNA fragment had a base sequence of SEQ ID NO: 55.

For deletion of the other region b1137-b1168, a linear DNA fragment containing a kanamycin-resistant gene (Km^{R}) was constructed using homology arm A having a base sequence of SEQ ID NO: 31, homology arm B having a base sequence of SEQ ID NO: 32 and homology arm C having a base sequence of SEQ ID NO: 33, and primers 1137A-F (SEQ ID NO: 34), 1137A-R (SEQ ID NO: 35), 1167B-F (SEQ ID NO: 36), 1167B-R (SEQ ID NO: 37), 1168C-F (SEQ ID NO: 38) and 1168C-R (SEQ ID NO: 39). The resulting linear DNA fragment had a base sequence of SEQ ID NO: 56. Base sequences of the primer as used herein are as follows.
**Primer b0980A-F** (SEQ ID NO: 25):
   5'-ACTGTTGGTGTGATTCATCTGCG-3'
**Primer b0980A-R** (SEQ ID NO: 26):
   5'-ACCCCAGGTATCGAACTGATTACCACATTGTTTCTGCTCCTTAG-3'
**Primer b1051B-F** (SEQ ID NO: 27):
**Primer b1051B-R** (SEQ ID NO: 28):
   5'-GATGACCATGTACGCAACGCTTG-3'
**Primer b1052C-F** (SEQ ID NO: 29):
   5'-AATCAGTTCGATACCTGGGGT-3'
**Primer b1052C-R** (SEQ ID NO: 30):
**Primer b1137A-F** (SEQ ID NO: 34):
   5'-CTGATGGATGGCGCTAAACTGCTG-3'
**Primer b1137A-R** (SEQ ID NO: 35):
   5'-CGCAGGATCTCTTCAGGCGTTGAAGATGTATGTGAAGGGGCCGC-3'
**Primer b1167B-F** (SEQ ID NO: 36):
   5'-GGCTGATCAGCTAGCCCATGGGTATGATCCAGCTCTGGTATTCCGCA-3'
**Primer b1167B-R** (SEQ ID NO: 37):
   5'- CCAATAATGTCATCCGCCACGCG-3'
**Primer b1168C-F** (SEQ ID NO: 38): 5'-CAACGCCTGAAGAGATCCTGCG-3'
**Primer b1168C-R** (SEQ ID NO: 39):
   5'-GCCGATCAACGTCTCATTTTCGCCAAAAATTCGGGTTGATTCTGGGTCTG-3'

As shown in FIG. 3a, in order to construct an *E. coli* mutant strain with deletions of the *E. coli* chromosomal regions b0980-b1052 and b1137-b1168, the b0980-b1052 region of *E. coli* was replaced with a linear DNA fragment (b0980-b1052+Cm^{R}) containing Cm^{R} as a selectable marker, analogously to Example 2, and then a second linear DNA fragment (b1137-b1168+Km^{R}) was also introduced to replace the b1137-b1168 region of the *E. coli* chromosome, analogously to Example 2. The *E. coli* mutants having chromosomal replacements with two linear DNA fragments exhibit chloramphenicol and kanamycin resistance due to the presence of Cm^{R} and Km^{R} genes on the linear DNA fragments, so these *E. coli* strains were selected in a medium supplemented with chloramphenicol and kanamycin.

Thereafter, the selectable marker genes Cm^{R} and Km^{R} remaining in the *E. coli* mutant strains with deletions of b0980-b1052 and b1137-b1168 were simultaneously deleted analogously to Example 2, thereby constructing a mutant strain from which the *E. coli* b0980-b1052 and b1137-b1168 were deleted. Deletion of the selected genomic regions was confirmed by PCR. In FIG. 3b, M represents a marker, Lanes 1 to 3 are intended to confirm the incorporation of linear DNA fragments into *E. coli,* wherein Lane 1 represents a non-introduced MG1655 strain (positive control), Lane 2 represents PCR results to confirm introduction of a DNA fragment A-C-Cm *sacB* I-S*ec*I-B constructed to delete the b0980-b1052 region, Lane 3 represents PCR results to confirm introduction of a DNA fragment A-C-Km *sac*B I-S*ec*I-B constructed to delete the b1137-b1168 region, Lane 4 represents PCR results to confirm I-S*ec*I-mediated deletion of a Cm *sac*B I-S*ec*I-B region after introduction of a DNA fragment A-C-Cm *sac*B I-*Sec*I-B constructed to delete the b0980-b1052 region, Lane 5 represents PCR results to confirm I-S*ec*I-mediated deletion of a Km *sac*B I-S*ec*I-B region after introduction of a DNA fragment A-C-Km *sac*B I-S*ec*I-B constructed to delete the b1137-b1168 region, and Lanes 6 and 7 represent PCR results for each region between b0980-b1052 and between b1137-b1168, conducted to confirm whether the b0980-b1052 and b1137-b1168 regions were deleted from the *E. coli* strain. Lane 6 is an *E. coli* strain with non-deletion of the b0980-b1052 and b1137-b1168 regions, *e.g*. MG1655 strain having no deletion of target genomic regions similar to the sample of Lane 1, thereby showing a band. On the other hand, Lane 7 exhibits no characteristic band due to deletion of b0980-b1052 and b1137-b1168. The efficiency of simultaneous deletion of specific genomic regions was nearly 95%.

FIG. 3a shows a markerless deletion process of inserting two linear DNA fragments containing different selectable markers into two specific genomic regions of *E. coli* and simultaneously deleting the specific genomic regions.

### Example 4: Deletion of specific chromosomal regions containing essential gene(s) from microbes

A useless genomic cluster containing essential gene(s) necessary for survival of microbes cannot be deleted with a conventional genomic deletion technique. The conventional gene deletion method of the essential gene-containing region was disadvantageously conducted including division of a deletion-targeted specific region into two genomic regions with respect to the essential gene and two deletion operations of the target region. In order to solve such a disadvantage of the conventional method, only the desired chromosomal region was deleted while retaining the essential gene, using the recombination vector constructed in Examples of the present invention.

For this purpose, a specific genomic region yecD-araF (b1867-b1901) of *E. coli* containing an essential gene *arg*S (b1876) encoding an arginyl-tRNA synthetase was selected. For deletion of the specific genomic region yecD-araF (b1867-b1901), a linear DNA fragment having a structure of [homology arm A (SEQ ID NO: 40)-E(argS)-homology arm C (SEQ ID NO:42)-Cm^{R}-sacB-I-*Sce*I-homology arm B (SEQ ID NO: 41)] as shown in FIG. 4a was constructed according to the method disclosed in Example 1. The resulting linear DNA fragment had a base sequence of SEQ ID NO: 57.

In order to replace the b1867-b1901 genomic region, the above-constructed DNA fragment was introduced into the *E*. *coli* strains. Then, the *E. coli* strains with a deletion of yecD-araF (b1867-b1901) containing *arg*S (b1876) were cultured and selected in a chloramphenicol-containing medium. According to the method disclosed in Example 2, desired strains, exhibiting traceless deletion of the specific chromosomal region through homologous recombination while removing the selectable marker that was contained in the linear DNA fragment, were successfully selected. FIG. 4a shows a process illustrating deletion of a specific genomic region containing an essential gene. Replacement and removal of the linear DNA fragment into/from the *E. coli* chromosome were confirmed by PCR, using primers specific for a gene of the specific region to be deleted. In FIG. 4b, M: Marker, Lane 1: MG1655 strain not transformed with the linear DNA fragment, Lane 2: MG1655 strain transformed with the linear DNA fragment, thus confirming whether the targeted genomic region of the strain was replaced with the linear DNA fragment, Lane 3: PCR results confirming I-*Sce*I-mediated deletion of a Cm^{R}-*sac*B-I-*Sce*I-homology arm B portion from a DNA fragment consisting of homology arm A-E(argS)-homology arm C-Cm^{R}-*sac*B-I-*Sce*I-homology arm B, Lane 4: MG1655 strain with non-deletion of the specific chromosomal region, which is a sample similar to that of Lane 1. Appearance of two bands is due to simultaneous PCR of two regions between b1867-b1901. Lane 5: MG1655 strain with deletion of the specific chromosomal region and exhibiting no characteristic band, thus representing that the specific chromosomal region was correctly deleted. Instead of carrying out two deletion steps, *i.e.* one operation at each side with respect to the essential gene, the aforesaid method of the present invention enables advantageously rapid and efficient deletion of the targeted specific region even with single-deletion operation. Base sequences of primers as used herein are as follows.
**Primer b1867A-F** (SEQ ID NO: 44):
   5'-TGCGCTGGTGGTGATCGATTTAC-3'
**Primer b1867A-R** (SEQ ID NO: 45):
   5'-TGGCACAGGGCAACAGGGTAAACGCGTTGAGGATCTCTTCCACG-3'
**Primer *arg*S-F** (SEQ ID NO: 46):
   5'-TTACCCTGTTGCCCTGTGCCA-3'
**Primer *arg*S-R** (SEQ ID NO: 47):
**Primer b1901C-F** (SEQ ID NO: 48):
   5'-CTCCATAGGAGAGCAATATCACATCGC-3'
**Primer b1901C-R** (SEQ ID NO: 49):
**Primer b1900B-F** (SEQ ID NO: 50):
   5'-GGCTGATCAGCTAGCCCATGGGTATGACCAGCATTGAGTTGGCAGCG-3'
**Primer b1900B-R** (SEQ ID NO: 51):
   5'-ATGTCACAATCCGCTATGGCGG-3'

As apparent from the above description, the recombinant vector for deletion of specific chromosomal regions in accordance with the present invention is capable of conveniently and rapidly achieving successive deletion of targeted specific genes with only one vector, as compared to conventional gene deletion methods involving the use of multiple vectors. In addition, the present invention enables prompt and efficient deletion of target genomic regions containing essential gene(s) in a single-step fashion.
## Claims

1. A recombinant vector for deletion of specific chromosomal regions, comprising an arabinose-inducible promoter (Pₐᵣₐ); a gene encoding a protein involved in lambda (λ)-red recombination; a rhamnose-inducible promoter (Pᵣₕₐ); and a gene encoding the I-*Sce*I endonuclease, wherein the vector has a base sequence of SEQ ID NO: 1 and is represented by a cleavage map of FIG. 1.

2. *Escherichia coli* transformed with the recombination vector of claim 1.

3. A method for deletion of specific chromosomal regions of a microbe using the recombination vector of claim 1, comprising the steps of:
1) preparing a linear DNA fragment containing homology arms A and B which are involved in λ-red recombination when they are introduced into a target microbe; a selectable marker; an I-SceI recognition site which is involved in homologous recombination for removal of the selectable marker; and a homology arm C which is involved in homologous recombination for removal of the selectable marker;
2) introducing the linear DNA fragment into a microbe transformed with the recombination vector of claim 1 to replace a specific locus of the microbial chromosome with the linear DNA fragment through λ-red recombination between the homology arms of the DNA fragment and the microbial chromosome regions homologous to the homology arms; and
3) culturing the specific chromosomal locus-replaced microbe in a rhamnose-containing medium to induce expression of the I-SceI endonuclease, such that homologous recombination between the homology arm C of the DNA fragment and the microbial chromosomal region homologous to the homology arm C is driven to remove the selectable marker,
wherein the homology arm A is a region homologous to 50 to 500bp of one end of the deletion target domain of a microbial chromosome and the homology arm B is a region homologous to 50 to 500bp of the other end of the deletion target domain of the microbial chromosome, and
the homology arm C is a region that is homologous to a 300-500bp region contiguous to either one of the microbial chromosome regions homologous to the homology arm A and homology arm B.

4. The method according to claim 3, wherein the method includes repeating Steps 1 and 2 to prepare a plurality of different linear DNA fragments and introducing the linear DNA fragments into microbes transformed with a recombination vector of claim 1 to delete a plurality of specific microbial chromosomal regions.

5. The method according to claim 3 or 4, wherein the selectable marker is at least one selected from the group consisting of a chloramphenicol-resistant gene having a base sequence of SEQ ID NO: 13, a kanamycin-resistant gene having a base sequence of SEQ ID NO: 14, and sacB.

6. The method according to claim 5, further comprising culturing the microbes in a sucrose-containing medium after the step of removing the selectable marker.

7. The method according to claim 3 or 4, wherein the homology arm A, the homology arm B and the homology arm C have base sequences of SEQ ID NO: 15, SEQ ID NO: 16 and SEQ ID NO: 17, respectively.

8. The method according to claim 3 or 4, wherein the homology arm A, the homology arm B and the homology arm C have base sequences of SEQ ID NO: 22, SEQ ID NO: 23 and SEQ ID NO: 24, respectively.

9. The method according to claim 3 or 4, wherein the homology arm A, the homology arm B and homology arm C have base sequences of SEQ ID NO: 31, SEQ ID NO: 32 and SEQ ID NO: 33, respectively.

10. The method according to claim 3, wherein the specific chromosomal region of the microbe contains a gene essential for survival of the microbe, and the linear DNA fragment further contains the essential survival gene between the homology arm A and the homology arm C.

11. The method according to claim 10, wherein the essential gene is *arg*S having a base sequence of SEQ ID NO: 43, and the homology arm A, the homology arm B and the homology arm C have base sequences of SEQ ID NO: 40, SEQ ID NO: 41 and SEQ ID NO: 42, respectively.

## Patentansprüche

1. Rekombinanter Vektor zur Deletion spezifischer Chromosomenregionen, aufweisend einen Arabinose-induzierbaren Promotor (Pₐᵣₐ); ein Gen, das ein Protein kodiert, welches bei einer Lambda (λ)-Rot-Rekombination beteiligt ist; einen Rhamnose-induzierbaren Promotor (Pᵣₕₐ); und ein Gen, das die I-*Sce*I-Endonuclease kodiert, wobei der Vektor eine Basensequenz von SEQ ID NO:1 aufweist und durch einen Spaltungsplan von Fig. 1 repräsentiert ist.

2. *Escherichia coli,* das mit dem Rekombinationsvektor nach Anspruch 1 transformiert ist.

3. Verfahren zur Deletion spezifischer Chromosomenregionen einer Mikrobe unter Verwendung des Rekombinationsvektors nach Anspruch 1, welches folgende Schritte umfasst:
1) Vorbereiten eines linearen DNA-Fragmentes, das enthält: Homologiearme A und B, die bei einer λ-Rot-Rekombination beteiligt sind, wenn sie in eine Zielmikrobe eingebracht werden; einen Selektionsmarker; einen I-Scel-Erkennungsort, der bei einer homologen Rekombination zum Entfernen des Selektionsmarkers beteiligt ist; und einen Homologiearm C, der bei einer homologen Rekombination zum Entfernen des Selektionsmarkers beteiligt ist;
2) Einbringen des linearen DNA-Fragmentes in eine Mikrobe, die mit dem Rekombinationsvektor nach Anspruch 1 transformiert ist, um einen spezifischen Ort des Mikrobenchromosoms mit dem linearen DNA-Fragment zu ersetzen, und zwar mittels einer λ-Rot-Rekombination zwischen den Homologiearmen des DNA-Fragmentes und den Mikrobenchromosomregionen, die zu den Homologiearmen homolog sind; und
3) Kultivieren der eine Ersetzung eines spezifischen Chromosomenortes aufweisenden Mikrobe in einem Rhamnose enthaltenden Medium, um eine Expression der I-Scel-Endonuclease zu induzieren, derart, dass eine homologe Rekombination zwischen dem Homologiearm C des DNA-Fragmentes und der Mikrobenchromosomenregion, die zu dem Homologiearm C homolog ist, angetrieben wird, um den Selektionsmarker zu entfernen,
wobei der Homologiearm A eine Region ist, die zu 50 bis 500bp von dem einen Ende der Deletionszieldomäne eines Mikrobenchromosoms homolog ist und der Homologiearm B eine Region ist, die zu 50 - 500bp des anderen Endes der Deletionszieldomäne des Mikrobenchromosoms homolog ist, und
der Homologiearm C eine Region ist, die homolog zu einer 300 bis 500bp aufweisenden Region ist, welche benachbart zu einer der Mikrobenchromosomregionen ist, die zu dem Homologiearm A und dem Homologiearm B homolog sind.

4. Verfahren nach Anspruch 3, wobei das Verfahren ein Wiederholen der Schritte 1 und 2 beinhaltet, um eine Mehrzahl von unterschiedlichen linearen DNA-Fragmenten vorzubereiten und die linearen DNA-Fragmente in Mikroben einzubringen, die mit einem Rekombinationsvektor nach Anspruch 1 transformiert sind, um eine Mehrzahl von spezifischen Mikrobenchromosomenregionen zu deletieren.

5. Verfahren nach Anspruch 3 oder 4, wobei der Selektionsmarker mindestens einer ist, der aus der Gruppe gewählt ist, die aus einem Chloramphenicol-resistenten Gen, das eine Basensequenz von SEQ ID NO:13 aufweist, einem Kanamycin-resistenten Gen, das eine Basensequenz von SEQ ID NO: 14 aufweist, und sacB besteht.

6. Verfahren nach Anspruch 5, das weiter beinhaltet, das die Mikroben in einem Sucrose enthaltenden Medium kultiviert werden, und zwar nach dem Schritt des Entfernens des Selektionsmarkers.

7. Verfahren nach Anspruch 3 oder 4, wobei der Homologiearm A, der Homologiearm B und der Homologiearm C Basensequenzen von SEQ ID NO: 15, SEQ ID NO: 16 bzw. SEQ ID NO: 17 aufweisen.

8. Verfahren nach Anspruch 3 oder 4, wobei der Homologiearm A, der Homologiearm B und der Homologiearm C Basensequenzen von SEQ ID NO: 22, SEQ ID NO: 23 bzw. SEQ ID NO: 24 aufweisen.

9. Verfahren nach Anspruch 3 oder 4, wobei der Homologiearm A, der Homologiearm B und der Homologiearm C Basensequenzen von SEQ ID NO: 31, SEQ ID NO: 32 bzw. SEQ ID NO: 33 aufweisen.

10. Verfahren nach Anspruch 3, wobei die spezifische Chromosomenregion der Mikrobe ein Gen enthält, das für das Überleben der Mikrobe essentiell ist und das lineare DNA-Fragment weiter das für ein Überleben essentielle Gen zwischen dem Homologiearm A und dem Homologiearm C enthält.

11. Verfahren nach Anspruch 10, wobei das essentielle Gen *arg*S ist, das eine Basensequenz von SEQ ID NO: 43 aufweist, und der Homologiearm A, der Homologiearm B und der Homologiearm C Basensequenzen von SEQ ID NO: 40, SEQ ID NO: 41 bzw. SEQ ID NO: 42 aufweisen.

## Revendications

1. Vecteur recombinant pour délétion de régions chromosomiques spécifiques, comprenant un promoteur inductible par l'arabinose (Para) ; un gène codant une protéine impliquée dans une recombinaison lambda (λ)-red ; un promoteur inductible par le rhamnose (Pᵣₕₐ) ; et un gène codant pour l'endonucléase I-*Sce*I, **caractérisé en ce que** le vecteur a une séquence de base de SEQ ID NO: 1 et est représenté par la cartographie de clivage de la Figure 1.

2. *Escherichia coli* transformé avec le vecteur de recombinaison de la revendication 1.

3. Procédé pour la délétion de régions chromosomiques spécifiques d'un microbe utilisant le vecteur de recombinaison de la revendication 1, comprenant les étapes consistant à :
1) préparer un fragment d'ADN linéaire contenant des bras d'homologie A et B qui sont impliqués dans la recombinaison λ-red lorsqu'ils sont introduits dans un microbe cible ; un marqueur sélectionnable ; un site de reconnaissance I*-SceI* qui est impliqué dans la recombinaison homologue pour élimination du marqueur sélectionnable ; et un bras d'homologie C qui est impliqué dans la recombinaison homologue pour l'élimination du marqueur sélectionnable,
2) introduire le fragment d'ADN linéaire dans un microbe transformé avec le vecteur de recombinaison de la revendication 1 pour remplacer un locus spécifique du chromosome microbien par le fragment d'ADN linéaire par recombinaison λ-red entre les bras d'homologie du fragment d'ADN et les régions du chromosome microbien homologues aux bras d'homologie ; et
3) mettre en culture le microbe après remplacement du locus chromosomique spécifique dans un milieu contenant du rhamnose pour induire l'expression de l'endonucléase I-*Sce*I, de sorte qu'une recombinaison homologue entre le bras d'homologie C du fragment d'ADN et la région chromosomique microbienne homologue au bras d'homologie C soit conduite pour éliminer le marqueur sélectionnable,
**caractérisé en ce que** le bras d'homologie A est une région homologue à 50 à 500 pb d'une extrémité du domaine cible de délétion d'un chromosome microbien et le bras d'homologie B est une région homologue à 50 à 500 pb de l'autre extrémité du domaine cible de délétion du chromosome microbien, et
le bras d'homologie C est une région qui est homologue à une région de 300 à 500 pb contiguë à l'une des régions de chromosome microbien homologue au bras d'homologie A et au bras d'homologie B.

4. Procédé selon la revendication 3, **caractérisé en ce que** le procédé comprend la répétition des étapes 1 et 2 pour préparer une pluralité de fragments d'ADN linéaire différents et introduire les fragments d'ADN linéaire dans des microbes transformés avec un vecteur de recombinaison de la revendication 1 pour déléter une pluralité de régions chromosomiques microbiennes spécifiques.

5. Procédé selon la revendication 3 ou 4, **caractérisé en ce que** le marqueur sélectionnable est au moins l'un choisi dans le groupe constitué d'un gène de résistance au chloramphénicol ayant une séquence de base de SEQ ID NO: 13, un gène de résistance à la kanamycine ayant une séquence de base de SEQ ID NO: 14, et sacB.

6. Procédé selon la revendication 5, comprenant en outre la mise en culture des microbes dans un milieu contenant du saccharose après l'étape consistant à éliminer le marqueur sélectionnable.

7. Procédé selon la revendication 3 ou 4, **caractérisé en ce que** le bras d'homologie A, le bras d'homologie B et le bras d'homologie C ont des séquences de base de SEQ ID NO: 15, SEQ ID NO: 16 et SEQ ID NO: 17, respectivement.

8. Procédé selon la revendication 3 ou 4, **caractérisé en ce que** le bras d'homologie A, le bras d'homologie B et le bras d'homologie C ont des séquences de base de SEQ ID NO: 22, SEQ ID NO: 23 et SEQ ID NO: 24, respectivement.

9. Procédé selon la revendication 3 ou 4, **caractérisé en ce que** le bras d'homologie A, le bras d'homologie B et le bras d'homologie C ont des séquences de base de SEQ ID NO: 31, SEQ ID NO: 32 et SEQ ID NO: 33, respectivement.

10. Procédé selon la revendication 3, **caractérisé en ce que** la région chromosomique spécifique du microbe contient un gène essentiel pour la survie du microbe, et le fragment d'ADN linéaire contient en outre le gène de survie essentiel entre le bras d'homologie A et le bras d'homologie C.

11. Procédé selon la revendication 10, **caractérisé en ce que** le gène essentiel est *arg*S ayant une séquence de base de SEQ ID NO: 43, et le bras d'homologie A, le bras d'homologie B et le bras d'homologie C ont des séquences de base de SEQ ID NO: 40, SEQ ID NO: 41 et SEQ ID NO: 42, respectivement.


<110> Korea Advanced Institute of Science and Technology
<120> A RECOMBINANT VECTOR FOR DELETING SPECIFIC REGIONS OF THE CHROMOSOME AND A METHOD FOR DELETING SPECIFIC CHROMOSOMAL REGIONS OF CHROMOSOME IN THE MICROORGANISM USING THE SAME
<160> 57
<170> Kopatent In 1.71
<210> 1
   <211> 9036
   <212> DNA
   <213> Artificial Sequence
<220>
   <223> pREDI vector
<400> 1
   catcgattta ttatgacaac ttgacggcta catcattcac tttttcttca caaccggcac      60
   ggaactcgct cgggctggcc ccggtgcatt ttttaaatac ccgcgagaaa tagagttgat     120
   cgtcaaaacc aacattgcga ccgacggtgg cgataggcat ccgggtggtg ctcaaaagca     180
   gcttcgcctg gctgatacgt tggtcctcgc gccagcttaa gacgctaatc cctaactgct     240
   ggcggaaaag atgtgacaga cgcgacggcg acaagcaaac atgctgtgcg acgctggcga     300
   tatcaaaatt gctgtctgcc aggtgatcgc tgatgtactg acaagcctcg cgtacccgat     360
   tatccatcgg tggatggagc gactcgttaa tcgcttccat gcgccgcagt aacaattgct     420
   caagcagatt tatcgccagc agctccgaat agcgcccttc cccttgcccg gcgttaatga     480
   tttgcccaaa caggtcgctg aaatgcggct ggtgcgcttc atccgggcga aagaaccccg     540
   tattggcaaa tattgacggc cagttaagcc attcatgcca gtaggcgcgc ggacgaaagt     600
   aaacccactg gtgataccat tcgcgagcct ccggatgacg accgtagtga tgaatctctc     660
   ctggcgggaa cagcaaaata tcacccggtc ggcaaacaaa ttctcgtccc tgatttttca      720
   ccaccccctg accgcgaatg gtgagattga gaatataacc tttcattccc agcggtcggt     780
   cgataaaaaa atcgagataa ccgttggcct caatcggcgt taaacccgcc accagatggg     840
   cattaaacga gtatcccggc agcaggggat cattttgcgc ttcagccata cttttcatac      900
   tcccgccatt cagagaagaa accaattgtc catattgcat cagacattgc cgtcactgcg     960
   tcttttactg gctcttctcg ctaaccaaac cggtaacccc gcttattaaa agcattctgt    1020
   aacaaagcgg gaccaaagcc atgacaaaaa cgcgtaacaa aagtgtctat aatcacggca    1080
   gaaaagtcca cattgattat ttgcacggcg tcacactttg ctatgccata gcatttttat    1140
   ccataagatt agcggatcct acctgacgct ttttatcgca actctctact gtttctccat    1200
   acccgttttt ttgggaattc gagctctaag gaggttataa aaaatggata ttaatactga    1260
   aactgagatc aagcaaaagc attcactaac cccctttcct gttttcctaa tcagcccggc    1320
   atttcgcggg cgatattttc acagctattt caggagttca gccatgaacg cttattacat     1380
   tcaggatcgt cttgaggctc agagctgggc gcgtcactac cagcagctcg cccgtgaaga    1440
   gaaagaggca gaactggcag acgacatgga aaaaggcctg ccccagcacc tgtttgaatc    1500
   gctatgcatc gatcatttgc aacgccacgg ggccagcaaa aaatccatta cccgtgcgtt    1560
   tgatgacgat gttgagtttc aggagcgcat ggcagaacac atccggtaca tggttgaaac    1620
   cattgctcac caccaggttg atattgattc agaggtataa aacgaatgag tactgcactc    1680
   gcaacgctgg ctgggaagct ggctgaacgt gtcggcatgg attctgtcga cccacaggaa 1740
   ctgatcacca ctcttcgcca gacggcattt aaaggtgatg ccagcgatgc gcagttcatc 1800
   gcattactga tcgttgccaa ccagtacggc cttaatccgt ggacgaaaga aatttacgcc 1860
   tttcctgata agcagaatgg catcgttccg gtggtgggcg ttgatggctg gtcccgcatc 1920
   atcaatgaaa accagcagtt tgatggcatg gactttgagc aggacaatga atcctgtaca 1980
   tgccggattt accgcaagga ccgtaatcat ccgatctgcg ttaccgaatg gatggatgaa 2040
   tgccgccgcg aaccattcaa aactcgcgaa ggcagagaaa tcacggggcc gtggcagtcg 2100
   catcccaaac ggatgttacg tcataaagcc atgattcagt gtgcccgtct ggccttcgga 2160
   tttgctggta tctatgacaa ggatgaagcc gagcgcattg tcgaaaatac tgcatacact 2220
   gcagaacgtc agccggaacg cgacatcact ccggttaacg atgaaaccat gcaggagatt 2280
   aacactctgc tgatcgccct ggataaaaca tgggatgacg acttattgcc gctctgttcc 2340
   cagatatttc gccgcgacat tcgtgcatcg tcagaactga cacaggccga agcagtaaaa 2400
   gctcttggat tcctgaaaca gaaagccgca gagcagaagg tggcagcatg acaccggaca 2460
   ttatcctgca gcgtaccggg atcgatgtga gagctgtcga acagggggat gatgcgtggc 2520
   acaaattacg gctcggcgtc atcaccgctt cagaagttca caacgtgata gcaaaacccc 2580
   gctccggaaa gaagtggcct gacatgaaaa tgtcctactt ccacaccctg cttgctgagg 2640
   tttgcaccgg tgtggctccg gaagttaacg ctaaagcact ggcctgggga aaacagtacg 2700
   agaacgacgc cagaaccctg tttgaattca cttccggcgt gaatgttact gaatccccga 2760
   tcatctatcg cgacgaaagt atgcgtaccg cctgctctcc cgatggttta tgcagtgacg 2820
   gcaacggcct tgaactgaaa tgcccgttta cctcccggga tttcatgaag ttccggctcg 2880
   gtggtttcga ggccataaag tcagcttaca tggcccaggt gcagtacagc atgtgggtga 2940
   cgcgaaaaaa tgcctggtac tttgccaact atgacccgcg tatgaagcgt gaaggcctgc 3000
   attatgtcgt gattgagcgg gatgaaaagt acatggcgag ttttgacgag atcgtgccgg 3060
   agttcatcga aaaaatggac gaggcactgg ctgaaattgg ttttgtattt ggggagcaat 3120
   ggcgatgacg catcctcacg ataatatccg ggtaggcgca atcactttcg tctactccgt 3180
   tacaaagcga ggctgggtat ttcccggcct ttctgttatc cgaaatccac tgaaagcaca 3240
   gcggctggct gaggagataa ataataaacg aggggctgta tgcacaaagc atcttctgtt 3300
   gagttaagaa cgagtatcga gatggcacat agccttgctc aaattggaat caggtttgtg 3360
   ccaataccag tagaaacaga cgaagaatcc atgggtcgac ttattatttc aggaaagttt 3420
   cggaggagat agtgttcggc agtttgtaca tcatctgcgg gatcaggtac ggtttgatca 3480
   ggttgtagaa gatcaggtaa gacatagaat cgatgtagat gatcggtttg tttttgttga 3540
   tttttacgta acagttcagt tggaatttgt tacgcagacc cttaaccagg tattctactt 3600
   cttcgaaagt gaaagactgg gtgttcagta cgatcgattt gttggtagag tttttgttgt 3660
   aatcccattt accaccatca tccatgaacc agtatgccag agacatcggg gtcaggtagt 3720
   tttcaaccag gttgttcggg atggtttttt tgttgttaac gatgaacagg ttagccagtt 3780
   tgttgaaagc ttggtgtttg aaagacagta cccactgatc gtacagcaga catacgtggt 3840
   ccatgtatgc tttgtttttc cactcgaact gcatacagta ggttttacct tcatcacgag 3900
   aacggatgta agcatcaccc aggatcagac cgatacctgc ttcgaactgt tcgatgttca 3960
   gttcgatcag ctgggatttg tattctttca gcagtttaga gttcggaccc aggttcatta 4020
   cctggttttt ttgatgcata tgtgatcctg ctgaatttca ttacgaccag tctaaaaagc 4080
   gcctgaattc gcgaccttct cgttactgac aggaaaatgg gccattggca accagggaaa 4140
   gatgaacgtg atgatgttca caatttgctg aattgtggtg atgtgatgct caccgcattt 4200
   cctgaaaatt cacgctgtat cttgaaaaat cgacgttttt tacgtggttt tccgtcgaaa 4260
   atttaaggta agaacctgac ctcgtgatta ctatttcgcc gtgttgacga catcaggagg 4320
   ccagtatgac cgtattacat agtgtggatt tttttccgtc tggtaacgcg tccgtggcga 4380
   tagaaccccg gctcccgcag gcggattttc ctgaacatca tcatgatttt catgaaattg 4440
   tgattgtcga acatggcacg ggtattcatg tgtttaatgg gcagccctat accatcaccg 4500
   gtggcacggt ctgtttcgta cgcgatcatg atcggcatct gtatgaacat accgataatc 4560
   tgtgtctgac caatgtgctg tatcgctcgc cggatcgatt tcagtttctc gccgggctga 4620
   atcagttgct gccacaagag ctggatgggc agtatccgtc tcactggcgc gttaaccaca 4680
   gcgtattgca gcaggtgcga cagctggttg cacagatgga acagcaggaa ggggaaaatg 4740
   atttaccctc gaccgccagt cgcgagatct tgtttatgca attactgctc ttgctgcgta 4800
   aaagcagttt gcaggagaac ctggaaaaca gcgcatcacg tctcaacttg cttctggcct 4860
   ggctggagga ccattttgcc gatgaggtga attgggatgc cgtggcggat caattttctc 4920
   tttcactgcg tacgctacat cggcagctta agcagcaaac gggactgacg cctcagcgat 4980
   acctgaaccg cctgcgactg atgaaagccc gacatctgct acgccacagc gaggccagcg 5040
   ttactgacat cgcctatcgc tgtggattca gcgacagtaa ccacttttcg acgctttttc 5100
   gccgagagtt taactggtca ccgcgtgata ttcgccaggg acgggatggc tttctgcaat 5160
   aacgcgaatc ttctcaacgt atttgtacgc catattgcga ataatcaact tcgttctctg 5220
   gccgaggtag ccacggtggc gcatcagtta aaacttctca aagatgattt ttttgccagc 5280
   gaccagcagg cagtcgctgt ggctgaccgt tatccgcaag atgtctttgc tgaacataca 5340
   catgattttt gtgagctggt gattgtctgg cgcggtaatg gcctgcatgt actcaacgat 5400
   cgcccttatc gcattacccg tggcgatctc ttttacattc atgctgacga taaacactcc 5460
   tacgcttccg ttaacgatct ggttttgcag aatattattt attgcccgga gcgtctgaag 5520
   ctgaatcttg actggcaggg ggcgattccg ggatttaacg ccagcgcagg gcaaccacac 5580
   tggcgcttag gtagcatggg gatggcgcag gcgcggcagg ttatcggtca gcttgagcat 5640
   gaaagtagtc agcatgtgcc gtttgctaac gaaatggctg agttgctgtt cgggcagttg 5700
   gtgatgttgc tgaatcgcca tcgttacacc agtgattcgt tgccgccaac atccagcgaa 5760
   acgttgctgg ataagctgat tacccggctg gcggctagcc tgaaaagtcc ctttgcgctg 5820
   gataaatttt gtgatgaggc atcgtgcagt gagcgcgttt tgcgtcagca atttcgccag 5880
   cagactggaa tgaccatcaa tcaatatctg cgacaggtca gagtgtgtca tgcgcaatat 5940
   cttctccagc atagccgcct gttaatcagt gatatttcga ccgaatgtgg ctttgaagat 6000
   agtaactatt tttcggtggt gtttacccgg gaaaccggga tgacgcccag ccagtggcgt 6060
   catctcaatt cgcagaaaga ttaattcgcc atgccccatg ggtatggaca gttttccctt 6120
   tgatatgtaa cggtgaacag ttgttctact tttgtttgtt agtcttgatg cttcactgat 6180
   agatacaaga gccataagaa cctcagatcc ttccgtattt agccagtatg ttctctagtg 6240
   tggttcgttg tttttgcgtg agccatgaga acgaaccatt gagatcatac ttactttgca 6300
   tgtcactcaa aaattttgcc tcaaaactgg tgagctgaat ttttgcagtt aaagcatcgt 6360
   gtagtgtttt tcttagtccg ttacgtaggt aggaatctga tgtaatggtt gttggtattt 6420
   tgtcaccatt catttttatc tggttgttct caagttcggt tacgagatcc atttgtctat 6480
   ctagttcaac ttggaaaatc aacgtatcag tcgggcggcc tcgcttatca accaccaatt 6540
   tcatattgct gtaagtgttt aaatctttac ttattggttt caaaacccat tggttaagcc 6600
   ttttaaactc atggtagtta ttttcaagca ttaacatgaa cttaaattca tcaaggctaa 6660
   tctctatatt tgccttgtga gtttcttttt gtgttagttc ttttaataac cactcataaa 6720
   tcctcataga gtatttgttt tcaaaagact taacatgttc cagattatat tttatgaatt 6780
   tttttaactg gaaaagataa ggcaatatct cttcactaaa aactaattct aatttttcgc 6840
   ttgagaactt ggcatagttt gtccactgga aaatctcaaa gcctttaacc aaaggattcc 6900
   tgatttccac agttctcgtc atcagctctc tggttgcttt agctaataca ccataagcat 6960
   tttccctact gatgttcatc atctgagcgt attggttata agtgaacgat accgtccgtt 7020
   ctttccttgt agggttttca atcgtggggt tgagtagtgc cacacagcat aaaattagct 7080
   tggtttcatg ctccgttaag tcatagcgac taatcgctag ttcatttgct ttgaaaacaa 7140
   ctaattcaga catacatctc aattggtcta ggtgatttta atcactatac caattgagat 7200
   gggctagtca atgataatta ctagtccttt tcctttgagt tgtgggtatc tgtaaattct 7260
   gctagacctt tgctggaaaa cttgtaaatt ctgctagacc ctctgtaaat tccgctagac 7320
   ctttgtgtgt tttttttgtt tatattcaag tggttataat ttatagaata aagaaagaat 7380
   aaaaaaagat aaaaagaata gatcccagcc ctgtgtataa ctcactactt tagtcagttc 7440
   cgcagtatta caaaaggatg tcgcaaacgc tgtttgctcc tctacaaaac agaccttaaa 7500
   accctaaagg cttaagtagc accctcgcaa gctcggttgc ggccgcaatc gggcaaatcg 7560
   ctgaatattc cttttgtctc cgaccatcag gcacctgagt cgctgtcttt ttcgtgacat 7620
   tcagttcgct gcgctcacgg ctctggcagt gaatgggggt aaatggcact acaggcgcct 7680
   tttatggatt catgcaagga aactacccat aatacaagaa aagcccgtca cgggcttctc 7740
   agggcgtttt atggcgggtc tgctatgtgg tgctatctga ctttttgctg ttcagcagtt 7800
   cctgccctct gattttccag tctgaccact tcggattatc ccgtgacagg tcattcagac 7860
   tggctaatgc acccagtaag gcagcggtat catcaacggg gtctgacgct cagtggaacg 7920
   aaaactcacg ttaagggatt ttggtcatga gattatcaaa aaggatcttc acctagatcc 7980
   ttttaaatta aaaatgaagt tttaaatcaa tctaaagtat atatgagtaa acttggtctg 8040
   acagttacca atgcttaatc agtgaggcac ctatctcagc gatctgtcta tttcgttcat 8100
   ccatagttgc ctgactcccc gtcgtgtaga taactacgat acgggagggc ttaccatctg 8160
   gccccagtgc tgcaatgata ccgcgagacc cacgctcacc ggctccagat ttatcagcaa 8220
   taaaccagcc agccggaagg gccgagcgca gaagtggtcc tgcaacttta tccgcctcca 8280
   tccagtctat taattgttgc cgggaagcta gagtaagtag ttcgccagtt aatagtttgc 8340
   gcaacgttgt tgccattgct acaggcatcg tggtgtcacg ctcgtcgttt ggtatggctt 8400
   cattcagctc cggttcccaa cgatcaaggc gagttacatg atcccccatg ttgtgcaaaa 8460
   aagcggttag ctccttcggt cctccgatcg ttgtcagaag taagttggcc gcagtgttat 8520
   cactcatggt tatggcagca ctgcataatt ctcttactgt catgccatcc gtaagatgct 8580
   tttctgtgac tggtgagtac tcaaccaagt cattctgaga atagtgtatg cggcgaccga 8640
   gttgctcttg cccggcgtca atacgggata ataccgcgcc acatagcaga actttaaaag 8700
   tgctcatcat tggaaaacgt tcttcggggc gaaaactctc aaggatctta ccgctgttga 8760
   gatccagttc gatgtaaccc actcgtgcac ccaactgatc ttcagcatct tttactttca 8820
   ccagcgtttc tgggtgagca aaaacaggaa ggcaaaatgc cgcaaaaaag ggaataaggg 8880
   cgacacggaa atgttgaata ctcatactct tcctttttca atattattga agcatttatc 8940
   agggttattg tctcatgagc ggatacatat ttgaatgtat ttagaaaaat aaacaaatag 9000
   gggttccgcg cacatttccc cgaaaagtgc cacctg 9036
<210> 2
   <211> 1233
   <212> DNA
   <213> Artificial Sequence
<220>
   <223> arabinose promoter
<400> 2
   ttatgacaac ttgacggcta catcattcac tttttcttca caaccggcac ggaactcgct 60
   cgggctggcc ccggtgcatt ttttaaatac ccgcgagaaa tagagttgat cgtcaaaacc 120
   aacattgcga ccgacggtgg cgataggcat ccgggtggtg ctcaaaagca gcttcgcctg 180
   gctgatacgt tggtcctcgc gccagcttaa gacgctaatc cctaactgct ggcggaaaag 240
   atgtgacaga cgcgacggcg acaagcaaac atgctgtgcg acgctggcga tatcaaaatt 300
   gctgtctgcc aggtgatcgc tgatgtactg acaagcctcg cgtacccgat tatccatcgg 360
   tggatggagc gactcgttaa tcgcttccat gcgccgcagt aacaattgct caagcagatt 420
   tatcgccagc agctccgaat agcgcccttc cccttgcccg gcgttaatga tttgcccaaa 480
   caggtcgctg aaatgcggct ggtgcgcttc atccgggcga aagaaccccg tattggcaaa 540
   tattgacggc cagttaagcc attcatgcca gtaggcgcgc ggacgaaagt aaacccactg 600
   gtgataccat tcgcgagcct ccggatgacg accgtagtga tgaatctctc ctggcgggaa 660
   cagcaaaata tcacccggtc ggcaaacaaa ttctcgtccc tgatttttca ccaccccctg 720
   accgcgaatg gtgagattga gaatataacc tttcattccc agcggtcggt cgataaaaaa 780
   atcgagataa ccgttggcct caatcggcgt taaacccgcc accagatggg cattaaacga 840
   gtatcccggc agcaggggat cattttgcgc ttcagccata cttttcatac tcccgccatt 900
   cagagaagaa accaattgtc catattgcat cagacattgc cgtcactgcg tcttttactg 960
   gctcttctcg ctaaccaaac cggtaacccc gcttattaaa agcattctgt aacaaagcgg 1020
   gaccaaagcc atgacaaaaa cgcgtaacaa aagtgtctat aatcacggca gaaaagtcca 1080
   cattgattat ttgcacggcg tcacactttg ctatgccata gcatttttat ccataagatt 1140
   agcggatcct acctgacgct ttttatcgca actctctact gtttctccat acccgttttt 1200
   ttgggaattc gagctctaag gaggttataa aaa 1233
<210> 3
   <211> 1885
   <212> DNA
   <213> Artificial Sequence
<220>
   <223> gamma-RED recombination System_gamma,beta,exo gene
<400> 3
   atggatatta atactgaaac tgagatcaag caaaagcatt cactaacccc ctttcctgtt      60
   ttcctaatca gcccggcatt tcgcgggcga tattttcaca gctatttcag gagttcagcc     120
   atgaacgctt attacattca ggatcgtctt gaggctcaga gctgggcgcg tcactaccag     180
   cagctcgccc gtgaagagaa agaggcagaa ctggcagacg acatggaaaa aggcctgccc     240
   cagcacctgt ttgaatcgct atgcatcgat catttgcaac gccacggggc cagcaaaaaa     300
   tccattaccc gtgcgtttga tgacgatgtt gagtttcagg agcgcatggc agaacacatc     360
   cggtacatgg ttgaaaccat tgctcaccac caggttgata ttgattcaga ggtataaaac     420
   gaatgagtac tgcactcgca acgctggctg ggaagctggc tgaacgtgtc ggcatggatt     480
   ctgtcgaccc acaggaactg atcaccactc ttcgccagac ggcatttaaa ggtgatgcca     540
   gcgatgcgca gttcatcgca ttactgatcg ttgccaacca gtacggcctt aatccgtgga     600
   cgaaagaaat ttacgccttt cctgataagc agaatggcat cgttccggtg gtgggcgttg     660
   atggctggtc ccgcatcatc aatgaaaacc agcagtttga tggcatggac tttgagcagg     720
   acaatgaatc ctgtacatgc cggatttacc gcaaggaccg taatcatccg atctgcgtta     780
   ccgaatggat ggatgaatgc cgccgcgaac cattcaaaac tcgcgaaggc agagaaatca     840
   cggggccgtg gcagtcgcat cccaaacgga tgttacgtca taaagccatg attcagtgtg     900
   cccgtctggc cttcggattt gctggtatct atgacaagga tgaagccgag cgcattgtcg     960
   aaaatactgc atacactgca gaacgtcagc cggaacgcga catcactccg gttaacgatg    1020
   aaaccatgca ggagattaac actctgctga tcgccctgga taaaacatgg gatgacgact    1080
   tattgccgct ctgttcccag atatttcgcc gcgacattcg tgcatcgtca gaactgacac    1140
   aggccgaagc agtaaaagct cttggattcc tgaaacagaa agccgcagag cagaaggtgg    1200
   cagcatgaca ccggacatta tcctgcagcg taccgggatc gatgtgagag ctgtcgaaca    1260
   gggggatgat gcgtggcaca aattacggct cggcgtcatc accgcttcag aagttcacaa    1320
   cgtgatagca aaaccccgct ccggaaagaa gtggcctgac atgaaaatgt cctacttcca    1380
   caccctgctt gctgaggttt gcaccggtgt ggctccggaa gttaacgcta aagcactggc    1440
   ctggggaaaa cagtacgaga acgacgccag aaccctgttt gaattcactt ccggcgtgaa    1500
   tgttactgaa tccccgatca tctatcgcga cgaaagtatg cgtaccgcct gctctcccga    1560
   tggtttatgc agtgacggca acggccttga actgaaatgc ccgtttacct cccgggattt    1620
   catgaagttc cggctcggtg gtttcgaggc cataaagtca gcttacatgg cccaggtgca    1680
   gtacagcatg tgggtgacgc gaaaaaatgc ctggtacttt gccaactatg acccgcgtat    1740
   gaagcgtgaa ggcctgcatt atgtcgtgat tgagcgggat gaaaagtaca tggcgagttt    1800
   tgacgagatc gtgccggagt tcatcgaaaa aatggacgag gcactggctg aaattggttt    1860
   tgtatttggg gagcaatggc gatga                                          1885
<210> 4
   <211> 417
   <212> DNA
   <213> Artificial Sequence
<220>
   <223> lambda-RED recombination System gamma gene
<400> 4
   atggatatta atactgaaac tgagatcaag caaaagcatt cactaacccc ctttcctgtt     60
   ttcctaatca gcccggcatt tcgcgggcga tattttcaca gctatttcag gagttcagcc    120
   atgaacgctt attacattca ggatcgtctt gaggctcaga gctgggcgcg tcactaccag    180
   cagctcgccc gtgaagagaa agaggcagaa ctggcagacg acatggaaaa aggcctgccc    240
   cagcacctgt ttgaatcgct atgcatcgat catttgcaac gccacggggc cagcaaaaaa    300
   tccattaccc gtgcgtttga tgacgatgtt gagtttcagg agcgcatggc agaacacatc    360
   cggtacatgg ttgaaaccat tgctcaccac caggttgata ttgattcaga ggtataa       417
<210> 5
   <211> 786
   <212> DNA
   <213> Artificial Sequence
<220>
   <223> lambda-RED recombination System beta gene
<400> 5
   atgagtactg cactcgcaac gctggctggg aagctggctg aacgtgtcgg catggattct     60
   gtcgacccac aggaactgat caccactctt cgccagacgg catttaaagg tgatgccagc    120
   gatgcgcagt tcatcgcatt actgatcgtt gccaaccagt acggccttaa tccgtggacg    180
   aaagaaattt acgcctttcc tgataagcag aatggcatcg ttccggtggt gggcgttgat    240
   ggctggtccc gcatcatcaa tgaaaaccag cagtttgatg gcatggactt tgagcaggac    300
   aatgaatcct gtacatgccg gatttaccgc aaggaccgta atcatccgat ctgcgttacc    360
   gaatggatgg atgaatgccg ccgcgaacca ttcaaaactc gcgaaggcag agaaatcacg    420
   gggccgtggc agtcgcatcc caaacggatg ttacgtcata aagccatgat tcagtgtgcc    480
   cgtctggcct tcggatttgc tggtatctat gacaaggatg aagccgagcg cattgtcgaa    540
   aatactgcat acactgcaga acgtcagccg gaacgcgaca tcactccggt taacgatgaa    600
   accatgcagg agattaacac tctgctgatc gccctggata aaacatggga tgacgactta    660
   ttgccgctct gttcccagat atttcgccgc gacattcgtg catcgtcaga actgacacag    720
   gccgaagcag taaaagctct tggattcctg aaacagaaag ccgcagagca gaaggtggca    780
   gcatga                                                               786
<210> 6
   <211> 681
   <212> DNA
   <213> Artificial Sequence
<220>
   <223> lambda-RED recombination System exo gene
<400> 6
   atgacaccgg acattatcct gcagcgtacc gggatcgatg tgagagctgt cgaacagggg      60
   gatgatgcgt ggcacaaatt acggctcggc gtcatcaccg cttcagaagt tcacaacgtg     120
   atagcaaaac cccgctccgg aaagaagtgg cctgacatga aaatgtccta cttccacacc     180
   ctgcttgctg aggtttgcac cggtgtggct ccggaagtta acgctaaagc actggcctgg     240
   ggaaaacagt acgagaacga cgccagaacc ctgtttgaat tcacttccgg cgtgaatgtt     300
   actgaatccc cgatcatcta tcgcgacgaa agtatgcgta ccgcctgctc tcccgatggt     360
   ttatgcagtg acggcaacgg ccttgaactg aaatgcccgt ttacctcccg ggatttcatg     420
   aagttccggc tcggtggttt cgaggccata aagtcagctt acatggccca ggtgcagtac     480
   agcatgtggg tgacgcgaaa aaatgcctgg tactttgcca actatgaccc gcgtatgaag     540
   cgtgaaggcc tgcattatgt cgtgattgag cgggatgaaa agtacatggc gagttttgac     600
   gagatcgtgc cggagttcat cgaaaaaatg gacgaggcac tggctgaaat tggttttgta     660
   tttggggagc aatggcgatg a                                               681
<210> 7
   <211> 2056
   <212> DNA
   <213> Artificial Sequence
<220>
   <223> rhamnose promoter
<400> 7
   ggcatggcga attaatcttt ctgcgaattg agatgacgcc actggctggg cgtcatcccg 60
   gtttcccggg taaacaccac cgaaaaatag ttactatctt caaagccaca ttcggtcgaa 120
   atatcactga ttaacaggcg gctatgctgg agaagatatt gcgcatgaca cactctgacc 180
   tgtcgcagat attgattgat ggtcattcca gtctgctggc gaaattgctg acgcaaaacg 240
   cgctcactgc acgatgcctc atcacaaaat ttatccagcg caaagggact tttcaggcta 300
   gccgccagcc gggtaatcag cttatccagc aacgtttcgc tggatgttgg cggcaacgaa 360
   tcactggtgt aacgatggcg attcagcaac atcaccaact gcccgaacag caactcagcc 420
   atttcgttag caaacggcac atgctgacta ctttcatgct caagctgacc gataacctgc 480
   cgcgcctgcg ccatccccat gctacctaag cgccagtgtg gttgccctgc gctggcgtta 540
   aatcccggaa tcgccccctg ccagtcaaga ttcagcttca gacgctccgg gcaataaata 600
   atattctgca aaaccagatc gttaacggaa gcgtaggagt gtttatcgtc agcatgaatg 660
   taaaagagat cgccacgggt aatgcgataa gggcgatcgt tgagtacatg caggccatta 720
   ccgcgccaga caatcaccag ctcacaaaaa tcatgtgtat gttcagcaaa gacatcttgc 780
   ggataacggt cagccacagc gactgcctgc tggtcgctgg caaaaaaatc atctttgaga 840
   agttttaact gatgcgccac cgtggctacc tcggccagag aacgaagttg attattcgca 900
   atatggcgta caaatacgtt gagaagattc gcgttattgc agaaagccat cccgtccctg 960
   gcgaatatca cgcggtgacc agttaaactc tcggcgaaaa agcgtcgaaa agtggttact 1020
   gtcgctgaat ccacagcgat aggcgatgtc agtaacgctg gcctcgctgt ggcgtagcag 1080
   atgtcgggct ttcatcagtc gcaggcggtt caggtatcgc tgaggcgtca gtcccgtttg 1140
   ctgcttaagc tgccgatgta gcgtacgcag tgaaagagaa aattgatccg ccacggcatc 1200
   ccaattcacc tcatcggcaa aatggtcctc cagccaggcc agaagcaagt tgagacgtga 1260
   tgcgctgttt tccaggttct cctgcaaact gcttttacgc agcaagagca gtaattgcat 1320
   aaacaagatc tcgcgactgg cggtcgaggg taaatcattt tccccttcct gctgttccat 1380
   ctgtgcaacc agctgtcgca cctgctgcaa tacgctgtgg ttaacgcgcc agtgagacgg 1440
   atactgccca tccagctctt gtggcagcaa ctgattcagc ccggcgagaa actgaaatcg 1500
   atccggcgag cgatacagca cattggtcag acacagatta tcggtatgtt catacagatg 1560
   ccgatcatga tcgcgtacga aacagaccgt gccaccggtg atggtatagg gctgcccatt 1620
   aaacacatga atacccgtgc catgttcgac aatcacaatt tcatgaaaat catgatgatg 1680
   ttcaggaaaa tccgcctgcg ggagccgggg ttctatcgcc acggacgcgt taccagacgg 1740
   aaaaaaatcc acactatgta atacggtcat actggcctcc tgatgtcgtc aacacggcga 1800
   aatagtaatc acgaggtcag gttcttacct taaattttcg acggaaaacc acgtaaaaaa 1860
   cgtcgatttt tcaagataca gcgtgaattt tcaggaaatg cggtgagcat cacatcacca 1920
   caattcagca aattgtgaac atcatcacgt tcatctttcc ctggttgcca atggcccatt 1980
   ttcctgtcag taacgagaag gtcgcgaatt caggcgcttt ttagactggt cgtaatgaaa 2040
   ttcagcagga tcacat 2056
<210> 8
   <211> 639
   <212> DNA
   <213> Artificial Sequence
<220>
   <223> I-Scel restriction enzyme
<400> R
<210> 9
   <211> 33
   <212> DNA
   <213> Artificial Sequence
<220>
   <223> primer Ncol-rha
<400> 9
   catgccatgg ggcatggcga attaatcttt ctg 33
<210> 10
   <211> 34
   <212> DNA
   <213> Artificial Sequence
<220>
   <223> primer prha
<400> 10
   atgtgatcct gctgaatttc attacgacca gtct 34
<210> 11
   <211> 69
   <212> DNA
   <213> Artificial Sequence
<220>
   <223> primer I-Scel-F
<400> 11
   ttagactggt cgtaatgaaa ttcagcagga tcacataatg catcaaaaaa accaggtaat 60
   gaacctggg 69
<210> 12
   <211> 48
   <212> DNA
   <213> Artificial Sequence
<220>
   <223> pr imer Ncol-I-Scel
<400> 12
   catgccatgg gtcgacttat tatttcagga aagtttcgga ggagatag 48
<210> 13
   <211> 660
   <212> DNA
   <213> Artificial Sequence
<220>
   <223> Chloramphenicol resistant gene
<400> 13
   atggagaaaa aaatcactgg atataccacc gttgatatat cccaatggca tcgtaaagaa 60
   cattttgagg catttcagtc agttgctcaa tgtacctata accagaccgt tcagctggat 120
   attacggcct ttttaaagac cgtaaagaaa aataagcaca agttttatcc ggcctttatt 180
   cacattcttg cccgcctgat gaatgctcat ccgaaattcc gtatggcaat gaaagacggt 240
   gagctggtga tatgggatag tgttcaccct tgttacaccg ttttccatga gcaaactgaa 300
   acgttttcat cgctctggag tgaataccac gacgatttcc ggcagtttct acacatatat 360
   tcgcaagatg tggcgtgtta cggtgaaaac ctggcctatt tccctaaagg gtttattgag 420
   aatatgtttt tcgtctcagc caatccctgg gtgagtttca ccagttttga tttaaacgtg 480
   gccaatatgg acaacttctt cgcccccgtt ttcaccatgg gcaaatatta tacgcaaggc 540
   gacaaggtgc tgatgccgct ggcgattcag gttcatcatg ccgtctgtga tggcttccat 600
   gtcggcagaa tgcttaatga attacaacag tactgcgatg agtggcaggg cggggcgtaa 660
<210> 14
   <211> 795
   <212> DNA
   <213> Artificial Sequence
<220>
   <223> Kanamycin resistant gene
<400> 14
   tcagaagaac tcgtcaagaa ggcgatagaa ggcgatgcgc tgcgaatcgg gagcggcgat 60
   accgtaaagc acgaggaagc ggtcagccca ttcgccgcca agctcttcag caatatcacg 120
   ggtagccaac gctatgtcct gatagcggtc cgccacaccc agccggccac agtcgatgaa 180
   tccagaaaag cggccatttt ccaccatgat attcggcaag caggcatcgc catgggtcac 240
   gacgagatcc tcgccgtcgc gccttgagcc tggcgaacag ttcggctggc gcgagcccct 300
   gatgctcttc gtccagatca tcctgatcga caagaccggc ttccatccga gtacgtgctc 360
   gctcgatgcg atgtttcgct tggtggtcga atgggcaggt agccggatca agcgtatgca 420
   gccgccgcat tgcatcagcc atgatggata ctttctcggc aggagcaagg tgagatgaca 480
   ggagatcctg ccccggcact tcgcccaata gcagccagtc ccttcccgct tcagtgacaa 540
   cgtcgagcac agctgcgcaa ggaacgcccg tcgtggccag ccacgatagc cgcgctgcct 600
   cgtcttggag ttcattcagg gcaccggaca ggtcggtctt gacaaaaaga accgggcgcc 660
   cctgcgctga cagccggaac acggcggcat cagagcagcc gattgtctgt tgtgcccagt 720
   catagccgaa tagcctctcc acccaagcgg ccggagaacc tgcgtgcaat ccatcttgtt 780
   caatcatcgg gcatc 795
<210> 15
   <211> 50
   <212> DNA
   <213> Artificial Sequence
<220>
   <223> homology arm A for b0004-b0024 deletion
<400> 15
   ccgccgattt tgctgcgttg cgtaaattga tgatgaatca tcagtaaaat 50
<210> 16
   <211> 50
   <212> DNA
   <213> Artificial Sequence
<220>
   <223> homology arm B for b0004-b0024 deletion
<400> 16
   ccgagtatac cttgtacagc ggtcaaggtt aaccggcgat tgagtaccga 50
<210> 17
   <211> 540
   <212> DNA
   <213> Artificial Sequence
<220>
   <223> homology arm C for b0004-b0024 deletion
<400> 17
<210> 18
   <211> 74
   <212> DNA
   <213> Artificial Sequence
<220>
   <223> primer b0004A-F
<400> 18
   ccgccgattt tgctgcgttg cgtaaattga tgatgaatca tcagtaaaat ccagacatga 60
   agctgatacg cggc 74
<210> 19
   <211> 54
   <212> DNA
   <213> Artificial Sequence
<220>
   <223> primer b0025C-R
<400> 19
   cgaattccgg tctccctata gtgagtcgta cagattgtca tccgcaaggg cctg 54
<210> 20
   <211> 30
   <212> DNA
   <213> Artificial Sequence
<220>
   <223> primer CSI-f
<400> 20
   tacgactcac tatagggaga ccggaattcg 30
<210> 21
   <211> 74
   <212> DNA
   <213> Artificial Sequence
<220>
   <223> primer b0024B-R
<400> 21
   ccgagtatac cttgtacagc ggtcaaggtt aaccggcgat tgagtaccga ctgcaggtcg 60
   actctagagg atct 74
<210> 22
   <211> 583
   <212> DNA
   <213> Artificial Sequence
<220>
   <223> homology arm A for b0980-b1052 deletion <400> 22
<210> 23
   <211> 553
   <212> DNA
   <213> Artificial Sequence
<220>
   <223> homology arm B for b0980-b1052 deletion
<400> 23
   agcattatta gtcgcactat accggggcag gggcatggca accaatatta tcccggtgtg 60
   caatgggatg tgcgtgactc cgcctggcgt tatgtcacga tccttgatct gccattctct 120
   ctggtttttg atactttact gctgccgatc gacattcatc atggcccgta tgagtgatta 180
   acgttcatcc cactcatcag ctgctgcgcg tccttcctcg gtatccagcg gtggctcaag 240
   ctgaaattcc ccctcgtccc attcatgtaa tgtattctct tcctgccact cctggcgtat 300
   ctctatctca tcatagtcgc catcaaaaac actttgcgcg gcttcaccgc taagcatagg 360
   taaacattca ccttcttccc cttcgtcggc aaaaaactca acttgccaca tgatgtcgcc 420
   gtcctgcaaa acgtattttt gggcattgaa ctgttgcaca ttcgcatctt cggcgtcgat 480
   gccggggttg tctgcaagaa attcttcgcg tgcagcgtca atggcttctt caagcgttgc 540
   gtacatggtc atc 553
<210> 24.
   <211> 509
   <212> DNA
   <213> Artificial Sequence
<220>
   <223> homology arm C for b0980-b1052 deletion
<400> 24
   aatcagttcg atacctgggg tattcgacga cgacgtagac tgttccatga atagactgcg 60
   ttgaataaca ctacgccagc ggtgacgaga aataccgctc tgaaaccgta gttcgctgaa 120
   atcgctgctc ccatcaatgg tccggtaacg ttgccaatat cacgaaacga ttggttatag 180
   ctgaagatac gcccggcgat ctggttgctc gagttgtaaa ccaacagtgt ctgtacggcg 240
   gggagtagtg caccatcggc ggcaccgagc aaaaaacgta aaatcccaag ttgcaatggc 300
   gtctgaacgt aagacattgg gatcaacagc agtacagaaa agatcagcgc tgtaatcagg 360
   atcttttcgg gtccgattcg atcgccaagt ttgccgagtc gtggtgcact tagcagagcc 420
   gccacgcctg gcaccgaggc gatcatgcca ctgataaagg cgacgttact gacgttaccc 480
   gccagttcgc ggacatacag cgtcagaat 509
<210> 25
   <211> 23
   <212> DNA
   <213> Artificial Sequence
<220>
   <223> primer b0980A-F
<400> 25
   actgttggtg tgattcatct gcg 23
<210> 26
   <211> 44
   <212> DNA
   <213> Artificial Sequence
<220>
   <223> primer b0980A-R
<400> 26
   accccaggta tcgaactgat taccacattg tttctgctcc ttag 44
<210> 27
   <211> 50
   <212> DNA
   <213> Artificial Sequence
<220>
   <223> primer b1051B-F
<400> 27
   ggctgatcag ctagcccatg ggtatgagca ttattagtcg cactataccg 50
<210> 28
   <211> 23
   <212> DNA
   <213> Artificial Sequence
<220>
   <223> primer b1051B-R
<400> 28
   gatgaccatg tacgcaacgc ttg 23
<210> 29
   <211> 21
   <212> DNA
   <213> Artificial Sequence
<220>
   <223> primer b1052C-F
<400> 29
   aatcagttcg atacctgggg t 21
<210> 30
   <211> 50
   <212> DNA
   <213> Artificial Sequence
<220>
   <223> primer b1052C-R
<400> 30
   gccgatcaac gtctcatttt cgccaaaaat tctgacgctg tatgtccgcg 50
<210> 31
   <211> 520
   <212> DNA
   <213> Artificial Sequence
<220>
   <223> homology arm A for b1137-b1158 deletion
<400> 31
   ctgatggatg gcgctaaact gctgaaatgt tcagagtttg gtgacgcgat catcgaaaac     60
   atgtaatgcc gtagtttgtt aaatttatta acgggagcgt aacgctcccg ttgttttttg     120
   ttaggctgct aacggttatc aaaattttat caaaaaaagt tatcaaaacc cctcggtagt    180
   tttggggtag gctggccggt caggtggtag ttctactact agtctcccac atagatattc    240
   cttagctttt tattattgct ggcggacgct cgttaatatt taaggtcttc attgattaag    300
   acatccccaa agttagttat gtattcactg ttattaggac aattatgaat taccactcct    360
   tacacccgct caaatattgt taaattgccg gttttgtatc aactactcac ccgggactcg    420
   ccaggggaca gccaacaggc attgggtgca atcaccttag cgttcaggta catgcggaat    480
   gtaaaaaagg ccgcgagcgc ggccccttca catacatctt                          520
<210> 32
   <211> 461
   <212> DNA
   <213> Artificial Sequence
<220>
   <223> homology arm B for b1137-b1158 deletion
<400> 32
   atccagctct ggtattccgc aaaagcagag tacctggcgg gagcgagata tgccgccaac     60
   aatatcaatc atatacttga agaagcgtca caagcgactc aaacagcggt taacattgcc    120
   gggaaggaat gcaacctcga ggagcaatat cagcttggca ctgaagcagc tctgaaacct    180
   cacctgcgca caatcatcat tctcaaacag ggaatagtct ggtgtacatc cctgcctggg    240
   aatcgggtcc tgttgtctcg tattcctgtt ttcccggaca gtaatttact gttggctcca    300
   gcaatcgaca ccgttaatag attacctatc ctgctctatc agaaccaatt tgcagatacg 360
   cgcattttgg ttacgataag cgatcagcat attcgtgggg cacttaatgt acccttgaaa 420
   ggggtaaggt atgtattacg cgtggcggat gacattattg g                        461
<210> 33
   <211> 583
   <212> DNA
   <213> Artificial Sequence
<220>
   <223> homology arm C for b1137-b1158 deletion
<400> 33
   caacgcctga agagatcctg cgacgggcaa taaataatgg ggagatcgtc cctttttacc       60
   aacctgtggt aaatggtcgg gaagggacat tgcggggagt tgaggtgtta gcccgctgga      120
   aacaacctca cggtggatat atatcacccg cggcatttat tccacttgct gaaaaatccg      180
   gattaatcgt tccgcttacg caaagcctga tgaatcaggt tgccagacag atgaacgcta      240
   tcgcgagtaa attgccggaa ggttttcata ttggaattaa ttttagcgct tcgcatatta      300
   tttcgccgac gtttgtcgac gagtgtttaa atttccgtga cagttttacc cgccgcgatt      360
   taaaccttgt tctggaagtc accgagcgtg agccattgaa tgttgatgaa agtctggttc      420
   agcggttgaa catactgcat gaaaatggtt ttgtcatcgc actggatgat ttcggtactg      480
   gctactcagg gctttcttat cttcatgacc tgcatattga ttatatcaaa attgatcaca      540
   gtttcgttgg ccgcgtaaac gcagacccag aatcaacccg aat                        583
<210> 34
   <211> 24
   <212> DNA
   <213> Artificial Sequence
<220>
   <223> primer b1137A-F
<400> 34
   ctgatggatg gcgctaaact gctg 24
<210> 35
   <211> 44
   <212> DNA
   <213> Artificial Sequence
<220>
   <223> primer b1137A-R
<400> 35
   cgcaggatct cttcaggcgt tgaagatgta tgtgaagggg ccgc 44
<210> 36
   <211> 47
   <212> DNA
   <213> Artificial Sequence
<220>
   <223> primer b1167B-F
<400> 36
   ggctgatcag ctagcccatg ggtatgatcc agctctggta ttccgca 47
<210> 37
   <211> 23
   <212> DNA
   <213> Artificial Sequence
<220>
   <223> primer b1167B-R
<400> 37
   ccaataatgt catccgccac gcg 23
<210> 38
   <211> 22
   <212> DNA
   <213> Artificial Sequence
<220>
   <223> primer b1168C-F
<400> 38
   caacgcctgaagagatcctgcg 22
<210> 39
   <211> 50
   <212> DNA
   <213> Artificial Sequence
<220>
   <223> primers b1168C-R
<400> 39
   gccgatcaacgtctcatttt cgccaaaaat tcgggttgat tctgggtctg 50
<210> 40
   <211> 536
   <212> DNA
   <213> Artificial Sequence
<220>
   <223> homology arm A for b1867-b1901 deletion
<400> 40
   tgcgctggtg gtgatcgatt tacaagaagg catcttacct tttgccggtg gtccacatac 60
   tgccgatgag gtagttaatc gcgccgggaa gctggcggcg aaatttcgcg ccagcggtca 120
   gcccgtgttt ctggtgcgcg ttggctggtc tgccgattac gctgaagcat taaaacagcc 180
   ggtcgatgcg ccctcaccgg ctaaagtgtt gcccgaaaat tggtggcaac atcctgctgc 240
   attaggtgca accgacagcg atatcgaaat catcaaacgt caatggggtg cgttttacgg 300
   tacggatctg gagttgcaat tacgccgccg aggtatcgat acaatagtgt tatgtgggat 360
   ctcgaccaat atcggtgttg aatccaccgc ccgcaatgcc tgggaacttg gctttaatct 420
   ggtgattgcc gaagacgcct gtagtgccgc cagcgccgag cagcacaata acagtatcaa 480
   ccatatctac ccgcgcatcg cccgtgtgcg tagcgtggaa gagatcctca acgcgt 536
<210> 41
   <211> 593
   <212> DNA
   <213> Artificial Sequence
<220>
   <223> homology arm B for b1867-b1901 deletion
<400> 41
   accagcattg agttggcagc gtcaaatgcc cccgggatgt cgttagattt ggtaggtacc      60
   tgataaattt gtttttccgg gaatccggcc gctttcagcg catccataga tcccgtagta     120
   cggcggcggg cggtatccag ttcgttggcg gtaatcgcca tcaccgcgct ttctttgaca     180
   tcccagccac gtttctgcat ctctttatac agttcctggc cctgacgttc gccaatttta     240
   gtcgccgcca tcatcaccag cggaacggta tccattggct tacctttggc gttaacaaac     300
   tggtcatcca cggcaatgac tttcatatcg tagccacgcg ctttcgcgac gatggcagag     360
   ccgagtttgg ggtccggagt acaaataacg aaaccttttg cgccactggc agccaggctg      420
   tcgatcgcgt tcaatgtttt ttcgccatcc ggcacggcaa tcttaataac ctcaaaccct     480
   aaatccttcc cggctttatc ggcaaacttc cattcggtct ggaaccacgg ctcttccggt     540
   tgcttcacca gaaaaccgag cttcaggttc tccgccatag cggattgtga cat            593
<210> 42
   <211> 564
   <212> DNA
   <213> Artificial Sequence
<220>
   <223> homology arm C for b1867-b1901 deletion
<400> 42
   ctccatagga gagcaatatc acatcgcaga attacagtga gaacgtgcat aaatttagcg      60
   ggaaaagaca taagggaaag ccaatttgtc agacaaattg tcgaatgcac agcagattaa     120
   tccataagat tagcctggaa atccttgttg tctttggtac ccatgcggga tgtcttcttt     180
   ttaaccagtc aataggccgc attacctggc gttgagtttt tgaaatggtg taataaccgc     240
   aactcaaaga tgtggaaaat gcacgtcatt catttcgtca ttaattatca ctgtgctcat     300
   taattaacag aacacgtata atgagagcca tctcgcaaaa atgaaaaaac gttttataaa     360
   atcatcactt catcatgaat tcaaattcat tgattaatat caacaagata caaaaagcac     420
   tatcattaaa attcattgca gttacattga tttcatcaat gaaatgtaaa aatatataaa     480
   cttgatgatt taagcatttt cttatacccg ttcagacgtt attcttattt cagatcatcg     540
   tcagaattga ctccacgatc acat                                            564
<210> 43
   <211> 1958
   <212> DNA
   <213> Artificial Sequence
<220>
   <223> argS gene
<400> 43
   ttaccctgtt gccctgtgcc aaccaaccgc tgatttcacg ccgcttctga tgcaatagtg 60
   aaaacggcaa tacgccacgc gcacgttgct gacgaaaaca gccatttgca gtatactccc 120
   gccctaattt ctttaactgg tgcgggcaat ttttgctcgc ttcatcaatg taaggtattc 180
   cggtgaatat tcaggctctt ctctcagaaa aagtccgtca ggccatgatt gcggcaggcg 240
   cgcctgcgga ttgcgaaccg caggttcgtc agtcagcaaa agttcagttc ggcgactatc 300
   aggctaacgg catgatggca gttgctaaaa aactgggtat ggcaccgcga caattagcag 360
   agcaggtgct gactcatctg gatcttaacg gtatcgccag caaagttgag atcgccggtc 420
   caggctttat caacattttc cttgatccgg cattcctggc tgaacatgtt cagcaggcgc 480
   tggcgtccga tcgtctcggt gttgctacgc cagaaaaaca gaccattgtg gttgactact 540
   ctgcgccaaa cgtggcgaaa gagatgcatg tcggtcacct gcgctctacc attattggtg 600
   acgcagcagt gcgtactctg gagttcctcg gtcacaaagt gattcgcgca aaccacgtcg 660
   gcgactgggg cactcagttc ggtatgctga ttgcatggct ggaaaagcag cagcaggaaa 720
   acgccggtga aatggagctg gctgaccttg aaggtttcta ccgcgatgcg aaaaagcatt 780
   acgatgaaga tgaagagttc gccgagcgcg cacgtaacta cgtggtaaaa ctgcaaagcg 840
   gtgacgaata tttccgcgag atgtggcgca aactggtcga catcaccatg acgcagaacc 900
   agatcaccta cgatcgtctc aacgtgacgc tgacccgtga tgacgtgatg ggcgaaagcc 960
   tctacaaccc gatgctgcca ggaattgtgg cggatctcaa agccaaaggt ctggcagtag 1020
   aaagcgaagg ggcgaccgtc gtattccttg atgagtttaa aaacaaggaa ggcgaaccga 1080
   tgggcgtgat cattcagaag aaagatggcg gctatctcta caccaccact gatatcgcct 1140
   gtgcgaaata tcgttatgaa acactgcatg ccgatcgcgt gctgtattac atcgactccc 1200
   gtcagcatca acacctgatg caggcatggg cgatcgtccg taaagcaggc tatgtaccgg 1260
   aatccgtacc gctggaacac cacatgttcg gcatgatgct gggtaaagac ggcaaaccgt 1320
   tcaaaacccg cgcgggtggt acagtgaaac tggccgatct gctggatgaa gccctggaac 1380
   gtgcacgccg tctggtggca gaaaagaacc cggatatgcc agccgacgag ctggaaaaac 1440
   tggctaacgc ggttggtatt ggtgcggtga aatatgcgga tctctccaaa aaccgcacca 1500
   cggactacat cttcgactgg gacaacatgc tggcgtttga gggtaatacc gcgccataca 1560
   tgcagtatgc atacacgcgt gtattgtccg tgttccgtaa agcagaaatt gacgaagagc 1620
   aactggctgc agctccggtt atcatccgtg aagatcgtga agcgcaactg gcagctcgcc 1680
   tgctgcagtt tgaagaaacc ctcaccgtgg ttgcccgtga aggcacgccg catgtaatgt 1740
   gtgcttacct gtacgatctg gccggtctgt tctctggctt ctacgagcac tgcccgatcc 1800
   tcagcgcaga aaacgaagaa gtgcgtaaca gccgtctaaa actggcacaa ctgacggcga 1860
   agacgctgaa gctgggtctg gatacgctgg gtattgagac tgtagagcgt atgtaatcga 1920
   tttttcgtga gagtgaagcc tgatcagggt tagccgat 1958
<210> 44
   <211> 23
   <212> DNA
   <213> Artificial Sequence
<220>
   <223> primer b1867A-F
<400> 44
   tgcgctggtg gtgatcgatt tac 23
<210> 45
   <211> 44
   <212> DNA
   <213> Artificial Sequence
<220>
   <223> primer b1867A-R
<400> 45
   tggcacaggg caacagggta aacgcgttga ggatctcttc cacg 44
<210> 46
   <211> 21
   <212>. DNA
   <213> Artificial Sequence
<220>
   <223> primer argS-F
<400> 46
   ttaccctgtt gccctgtgcca 21
<210> 47
   <211> 51
   <212> DNA
   <213> Artificial Sequence
<220>
   <223> primer argS-R
<400> 47
   gcgatgtgat attgctctcc tatggagatcggctaaccct gatcaggcttc 51
<210> 48
   <211> 27
   <212> DNA
   <213> Artificial Sequence
<220>
   <223> primer b1901C-F
<400> 48
   ctccatagga gagcaatatc acatcgc 27
<210> 49
   <211> 54
   <212> DNA
   <213> Artificial Sequence
<220>
   <223> primer b1901c-R
<400> 49
   gccgatcaac gtctcatttt cgccaaaaat gtgatcgtgg agtcaattct gacg 54
<210> 50
   <211> 47
   <212> DNA
   <213> Artificial Sequence
<220>
   <223> primer b1900B-F
<400> 50
   ggctgatcag ctagcccatg ggtatgacca gcattgagtt ggcagcg 47
<210> 51
   <211> 22
   <212> DNA
   <213> Artificial Sequence
<220>
   <223> primer b1900B-R
<400> 51
   atgtcacaat ccgctatggc gg 22
<210> 52
   <211> 18
   <212> DNA
   <213> Artificial Sequence
<220>
   <223> I-Scel restriction enzyme site
<400> 52
   tagggataac agggtaat 18
<210> 53
   <211> 1429
   <212> DNA
   <213> Artificial Sequence
<220>
   <223> sacB gene
<400> 53
   ttatttgtta actgttaatt gtccttgttc aaggatgctg tctttgacaa cagatgtttt 60
   cttgcctttg atgttcagca ggaagctcgg cgcaaacgtt gattgtttgt ctgcgtagaa 120
   tcctctgttt gtcatatagc ttgtaatcac gacattgttt cctttcgctt gaggtacagc 180
   gaagtgtgag taagtaaagg ttacatcgtt aggatcaaga tccattttta acacaaggcc 240
   agttttgttc agcggcttgt atgggccagt taaagaatta gaaacataac caagcatgta 300
   aatatcgtta gacgtaatgc cgtcaatcgt catttttgat ccgcgggagt cagtgaacag 360
   ataccatttg ccgttcattt taaagacgtt cgcgcgttca atttcatctg ttactgtgtt 420
   agatgcaatc agcggtttca tcactttttt cagtgtgtaa tcatcgttta gctcaatcat 480
   accgagagcg ccgtttgcta actcagccgt gcgtttttta tcgctttgca gaagtttttg 540
   actttcttga cggaagaatg atgtgctttt gccatagtat gctttgttaa ataaagattc 600
   ttcgccttgg tagccatctt cagttccagt gtttgcttca aatactaagt atttgtggcc 660
   tttatcttct acgtagtgag gatctctcag cgtatggttg tcgcctgagc tgtagttgcc 720
   ttcatcgatg aactgctgta cattttgata cgtttttccg tcaccgtcaa agattgattt 780
   ataatcctct acaccgttga tgttcaaaga gctgtctgat gctgatacgt taacttgtgc 840
   agttgtcagt gtttgtttgc cgtaatgttt accggagaaa tcagtgtaga ataaacggat 900
   ttttccgtca gatgtaaatg tggctgaacc tgaccattct tgtgtttggt cttttaggat 960
   agaatcattt gcatcgaatt tgtcgctgtc tttaaagacg cggccagcgt ttttccagct 1020
   gtcaatagaa gtttcgccga ctttttgata gaacatgtaa atcgatgtgt catccgcatt 1080
   tttaggatct ccggctaatg caaagacgat gtggtagccg tgatagtttg cgacagtgcc 1140
   gtcagcgttt tgtaatggcc agctgtccca aacgtccagg ccttttgcag aagagatatt 1200
   tttaattgtg gacgaatcaa attcaggaac ttgatatttt tcattttttt gctgttcagg 1260
   gatttgcagc atatcatggc gtgtaatatg ggaaatgccg tatgtttcct tatatggctt 1320
   ttggttcgtt tctttcgcaa acgcttgagt tgcgcctcct gccagcagtg cggtagtaaa 1380
   ggttaatact gttgcttgtt ttgcaaactt tttgatgttc atcgttcat 1429
<210> 54
   <211> 3535
   <212> DNA
   <213> Artificial Sequence
<220>
   <223> b0004-b0024
<400> 54
   ccgccgattt tgctgcgttg cgtaaattga tgatgaatca tcagtaaaat ccagacatga 60
   agctgatacg cggcatacat aatctcagcc aggccccgca agaagggtgt gtgctgacta 120
   ttggtaattt cgacggcgtg catcgcggtc atcgcgcgct gttacagggc ttgcaggaag 180
   aagggcgcaa gcgcaactta ccggtgatgg tgatgctttt tgaacctcaa ccactggaac 240
   tgtttgctac cgataaagcc ccggcaagac tgacccggct gcgggaaaaa ctgcgttacc 300
   ttgcagagtg tggcgttgat tacgtgctgt gcgtgcgttt cgacaggcgt ttcgcggcgt 360
   taaccgcgca aaatttcatc agcgatcttc tggtgaagca tttgcgcgta aaatttcttg 420
   ccgtaggtga tgatttccgc tttggcgctg gtcgtgaagg cgatttcttg ttattacaga 480
   aagctggcat ggaatacggc ttcgatatca ccagtacgca aactttttgc gaaggtggcg 540
   tgcgcatcag cagcaccgcc gtgcgtcagg cccttgcgga tgacaatctg tacgactcac 600
   tatagggaga ccggaattcg agctcggtac cttttggcga aaatgagacg ttgatcggca 660
   cgtaagaggt tccagctttc accataatga aataagatca ctaccgggcg tattttttga 720
   gttatcgaga ttttcaggag ctaaggaagc taaaatggag aaaaaaatca ctggatatac 780
   caccgttgat atatcccaat ggcatcgtaa agaacatttt gaggcatttc agtcagttgc 840
   tcaatgtacc tataaccaga ccgttcagct ggatattacg gcctttttaa agaccgtaaa 900
   gaaaaataag cacaagtttt atccggcctt tattcacatt cttgcccgcc tgatgaatgc 960
   tcatccgaaa ttccgtatgg caatgaaaga cggtgagctg gtgatatggg atagtgttca 1020
   cccttgttac accgttttcc atgagcaaac tgaaacgttt tcatcgctct ggagtgaata 1080
   ccacgacgat ttccggcagt ttctacacat atattcgcaa gatgtggcgt gttacggtga 1140
   aaacctggcc tatttcccta aagggtttat tgagaatatg tttttcgtct cagccaatcc 1200
   ctgggtgagt ttcaccagtt ttgatttaaa cgtggccaat atggacaact tcttcgcccc 1260
   cgttttcacc atgggcaaat attatacgca aggcgacaag gtgctgatgc cgctggcgat 1320
   tcaggttcat catgccgtct gtgatggctt ccatgtcggc agaatgctta atgaattaca 1380
   acagtactgc gatgagtggc agggcggggc gtaatttttt taaggcagtt attggtgcct 1440
   cactgattaa gcattggtaa ctgtcagatc cggggaattt atgggattca cctttatgtt 1500
   gataagaaat aaaagaaaat gccaatagga tatcggcatt ttcttttgcg tttttatttg 1560
   ttaactgtta attgtccttg ttcaaggatg ctgtctttga caacagatgt tttcttgcct 1620
   ttgatgttca gcaggaagct cggcgcaaac gttgattgtt tgtctgcgta gaatcctctg 1680
   tttgtcatat agcttgtaat cacgacattg tttcctttcg cttgaggtac agcgaagtgt 1740
   gagtaagtaa aggttacatc gttaggatca agatccattt ttaacacaag gccagttttg 1800
   ttcagcggct tgtatgggcc agttaaagaa ttagaaacat aaccaagcat gtaaatatcg 1860
   ttagacgtaa tgccgtcaat cgtcattttt gatccgcggg agtcagtgaa cagataccat 1920
   ttgccgttca ttttaaagac gttcgcgcgt tcaatttcat ctgttactgt gttagatgca 1980
   atcagcggtt tcatcacttt tttcagtgtg taatcatcgt ttagctcaat cataccgaga 2040
   gcgccgtttg ctaactcagc cgtgcgtttt ttatcgcttt gcagaagttt ttgactttct 2100
   tgacggaaga atgatgtgct tttgccatag tatgctttgt taaataaaga ttcttcgcct 2160
   tggtagccat cttcagttcc agtgtttgct tcaaatacta agtatttgtg gcctttatct 2220
   tctacgtagt gaggatctct cagcgtatgg ttgtcgcctg agctgtagtt gccttcatcg 2280
   atgaactgct gtacattttg atacgttttt ccgtcaccgt caaagattga tttataatcc 2340
   tctacaccgt tgatgttcaa agagctgtct gatgctgata cgttaacttg tgcagttgtc 2400
   agtgtttgtt tgccgtaatg tttaccggag aaatcagtgt agaataaacg gatttttccg 2460
   tcagatgtaa atgtggctga acctgaccat tcttgtgttt ggtcttttag gatagaatca 2520
   tttgcatcga atttgtcgct gtctttaaag acgcggccag cgtttttcca gctgtcaata 2580
   gaagtttcgc cgactttttg atagaacatg taaatcgatg tgtcatccgc atttttagga 2640
   tctccggcta atgcaaagac gatgtggtag ccgtgatagt ttgcgacagt gccgtcagcg 2700
   ttttgtaatg gccagctgtc ccaaacgtcc aggccttttg cagaagagat atttttaatt 2760
   gtggacgaat caaattcagg aacttgatat ttttcatttt tttgctgttc agggatttgc 2820
   agcatatcat ggcgtgtaat atgggaaatg ccgtatgttt ccttatatgg cttttggttc 2880
   gtttctttcg caaacgcttg agttgcgcct cctgccagca gtgcggtagt aaaggttaat 2940
   actgttgctt gttttgcaaa ctttttgatg ttcatcgttc atgtctcctt ttttatgtac 3000
   tgtgttagcg gtctgcttct tccagccctc ctgtttgaag atggcaagtt agttacgcac 3060
   aataaaaaaa gacctaaaat atgtaagggg tgacgccaaa gtatacactt tgccctttac 3120
   acattttagg tcttgcctgc tttatcagta acaaacccgc gcgatttact tttcgacctc 3180
   attctattag actctcgttt ggattgcaac tggtctattt tcctcttttg tttgatagaa 3240
   aatcataaaa ggatttgcag actacgggcc taaagaacta aaaaatctat ctgtttcttt 3300
   tcattctctg tattttttat agtttctgtt gcatgggcat aaagttgcct ttttaatcac 3360
   aattcagaaa atatcataat atctcatttc actaaataat agtgaacggc aggtatatgt 3420
   gatgggttaa aaaggatccg ctagggataa cagggtaata tagatcctct agagtcgacc 3480
   tgcagtcggt actcaatcgc cggttaacct tgaccgctgt acaaggtata ctcgg 3535
<210> 55
   <211> 4543
   <212> DNA
   <213> Artificial Sequence
<220>
   <223> b0980-b1052
<400> 55
   actgttggtg tgattcatct gcgttcacag ctggcgacca aacgcgctgc actgttggtg 60
   atgctgtgct ttttgctggc gggttactgg ctgtgggtcg gtattgatgg ctttgtactg 120
   ctcgcccagg atgctaacgg tccttccaat ccgttaatga aactggtggc agtgctacct 180
   ggtgcctgga tgaataattt tgtcgagtcg cccgttttgt ggatcttccc gctgctggga 240
   ttcttctgcc cattgctgac ggtgatggcg atttatcgtg gtcgcccggg ttggggattt 300
   ttgatggcat cattgatgca atttggcgtg attttcacgg caggcatcac gctgttcccc 360
   tttgtcatgc cgtcaagcgt gagtccgatc tccagcctga cgttgtggga cagtacttcc 420
   agtcagctga cgctgagcat tatgttggta atcgtgctga tatttttgcc cattgtgttg 480
   ctctacactc tctggagcta ctacaaaatg tgggggcgca tgacaacaga aactctccgc 540
   cgtaacgaaa acgagttgta ctaaggagca gaaacaatgt ggtaatcagt tcgatacctg 600
   gggtattcga cgacgacgta gactgttcca tgaatagact gcgttgaata acactacgcc 660
   agcggtgacg agaaataccg ctctgaaacc gtagttcgct gaaatcgctg ctcccatcaa 720
   tggtccggta acgttgccaa tatcacgaaa cgattggtta tagctgaaga tacgcccggc 780
   gatctggttg ctcgagttgt aaaccaacag tgtctgtacg gcggggagta gtgcaccatc 840
   ggcggcaccg agcaaaaaac gtaaaatccc aagttgcaat ggcgtctgaa cgtaagacat 900
   tgggatcaac agcagtacag aaaagatcag cgctgtaatc aggatctttt cgggtccgat 960
   tcgatcgcca agtttgccga gtcgtggtgc acttagcaga gccgccacgc ctggcaccga 1020
   ggcgatcatg ccactgataa aggcgacgtt actgacgtta cccgccagtt cgcggacata 1080
   cagcgtcaga atttttggcg aaaatgagac gttgatcggc acgtaagagg ttccagcttt 1140
   caccataatg aaataagatc actaccgggc gtattttttg agttatcgag attttcagga 1200
   gctaaggaag ctaaaatgga gaaaaaaatc actggatata ccaccgttga tatatcccaa 1260
   tggcatcgta aagaacattt tgaggcattt cagtcagttg ctcaatgtac ctataaccag 1320
   accgttcagc tggatattac ggccttttta aagaccgtaa agaaaaataa gcacaagttt 1380
   tatccggcct ttattcacat tcttgcccgc ctgatgaatg ctcatccgaa attccgtatg 1440
   gcaatgaaag acggtgagct ggtgatatgg gatagtgttc acccttgtta caccgttttc 1500
   catgagcaaa ctgaaacgtt ttcatcgctc tggagtgaat accacgacga tttccggcag 1560
   tttctacaca tatattcgca agatgtggcg tgttacggtg aaaacctggc ctatttccct 1620
   aaagggttta ttgagaatat gtttttcgtc tcagccaatc cctgggtgag tttcaccagt 1680
   tttgatttaa acgtggccaa tatggacaac ttcttcgccc ccgttttcac catgggcaaa 1740
   tattatacgc aaggcgacaa ggtgctgatg ccgctggcga ttcaggttca tcatgccgtc 1800
   tgtgatggct tccatgtcgg cagaatgctt aatgaattac aacagtactg cgatgagtgg 1860
   cagggcgggg cgtaattttt ttaaggcagt tattggtgcc tcactgatta agcattggta 1920
   actgtcagat ccggggaatt tatgggattc acctttatgt tgataagaaa taaaagaaaa 1980
   tgccaatagg atatcggcat tttcttttgc gtttttattt gttaactgtt aattgtcctt 2040
   gttcaaggat gctgtctttg acaacagatg ttttcttgcc tttgatgttc agcaggaagc 2100
   tcggcgcaaa cgttgattgt ttgtctgcgt agaatcctct gtttgtcata tagcttgtaa 2160
   tcacgacatt gtttcctttc gcttgaggta cagcgaagtg tgagtaagta aaggttacat 2220
   cgttaggatc aagatccatt tttaacacaa ggccagtttt gttcagcggc ttgtatgggc 2280
   cagttaaaga attagaaaca taaccaagca tgtaaatatc gttagacgta atgccgtcaa 2340
   tcgtcatttt tgatccgcgg gagtcagtga acagatacca tttgccgttc attttaaaga 2400
   cgttcgcgcg ttcaatttca tctgttactg tgttagatgc aatcagcggt ttcatcactt 2460
   ttttcagtgt gtaatcatcg tttagctcaa tcataccgag agcgccgttt gctaactcag 2520
   ccgtgcgttt tttatcgctt tgcagaagtt tttgactttc ttgacggaag aatgatgtgc 2580
   ttttgccata gtatgctttg ttaaataaag attcttcgcc ttggtagcca tcttcagttc 2640
   cagtgtttgc ttcaaatact aagtatttgt ggcctttatc ttctacgtag tgaggatctc 2700
   tcagcgtatg gttgtcgcct gagctgtagt tgccttcatc gatgaactgc tgtacatttt 2760
   gatacgtttt tccgtcaccg tcaaagattg atttataatc ctctacaccg ttgatgttca 2820
   aagagctgtc tgatgctgat acgttaactt gtgcagttgt cagtgtttgt ttgccgtaat 2880
   gtttaccgga gaaatcagtg tagaataaac ggatttttcc gtcagatgta aatgtggctg 2940
   aacctgacca ttcttgtgtt tggtctttta ggatagaatc atttgcatcg aatttgtcgc 3000
   tgtctttaaa gacgcggcca gcgtttttcc agctgtcaat agaagtttcg ccgacttttt 3060
   gatagaacat gtaaatcgat gtgtcatccg catttttagg atctccggct aatgcaaaga 3120
   cgatgtggta gccgtgatag tttgcgacag tgccgtcagc gttttgtaat ggccagctgt 3180
   cccaaacgtc caggcctttt gcagaagaga tatttttaat tgtggacgaa tcaaattcag 3240
   gaacttgata tttttcattt ttttgctgtt cagggatttg cagcatatca tggcgtgtaa 3300
   tatgggaaat gccgtatgtt tccttatatg gcttttggtt cgtttctttc gcaaacgctt 3360
   gagttgcgcc tcctgccagc agtgcggtag taaaggttaa tactgttgct tgttttgcaa 3420
   actttttgat gttcatcgtt catgtctcct tttttatgta ctgtgttagc ggtctgcttc 3480
   ttccagccct cctgtttgaa gatggcaagt tagttacgca caataaaaaa agacctaaaa 3540
   tatgtaaggg gtgacgccaa agtatacact ttgcccttta cacattttag gtcttgcctg 3600
   ctttatcagt aacaaacccg cgcgatttac ttttcgacct cattctatta gactctcgtt 3660
   tggattgcaa ctggtctatt ttcctctttt gtttgataga aaatcataaa aggatttgca 3720
   gactacgggc ctaaagaact aaaaaatcta tctgtttctt ttcattctct gtatttttta 3780
   tagtttctgt tgcatgggca taaagttgcc tttttaatca caattcagaa aatatcataa 3840
   tatctcattt cactaaataa tagtgaacgg caggtatatg tgatgggtta aaaaggatcc 3900
   gctagggata acagggtaat atagatcctc tagagtcgac ctgcaggcat gcaagcttgg 3960
   cactggctga tcagctagcc catgggtatg agcattatta gtcgcactat accggggcag 4020
   gggcatggca accaatatta tcccggtgtg caatgggatg tgcgtgactc cgcctggcgt 4080
   tatgtcacga tccttgatct gccattctct ctggtttttg atactttact gctgccgatc 4140
   gacattcatc atggcccgta tgagtgatta acgttcatcc cactcatcag ctgctgcgcg 4200
   tccttcctcg gtatccagcg gtggctcaag ctgaaattcc ccctcgtccc attcatgtaa 4260
   tgtattctct tcctgccact cctggcgtat ctctatctca tcatagtcgc catcaaaaac 4320
   actttgcgcg gcttcaccgc taagcatagg taaacattca ccttcttccc cttcgtcggc 4380
   aaaaaactca acttgccaca tgatgtcgcc gtcctgcaaa acgtattttt gggcattgaa 4440
   ctgttgcaca ttcgcatctt cggcgtcgat gccggggttg tctgcaagaa attcttcgcg 4500
   tgcagcgtca atggcttctt caagcgttgc gtacatggtc atc 4543
<210> 56
   <211> 4597
   <212> DNA
   <213> Artificial Sequence
<220>
   <223> b1137-b1158
<400> 56
   ctgatggatg gcgctaaact gctgaaatgt tcagagtttg gtgacgcgat catcgaaaac 60
   atgtaatgcc gtagtttgtt aaatttatta acgggagcgt aacgctcccg ttgttttttg 120
   ttaggctgct aacggttatc aaaattttat caaaaaaagt tatcaaaacc cctcggtagt 180
   tttggggtag gctggccggt caggtggtag ttctactact agtctcccac atagatattc 240
   cttagctttt tattattgct ggcggacgct cgttaatatt taaggtcttc attgattaag 300
   acatccccaa agttagttat gtattcactg ttattaggac aattatgaat taccactcct 360
   tacacccgct caaatattgt taaattgccg gttttgtatc aactactcac ccgggactcg 420
   ccaggggaca gccaacaggc attgggtgca atcaccttag cgttcaggta catgcggaat 480
   gtaaaaaagg ccgcgagcgc ggccccttca catacatctt caacgcctga agagatcctg 540
   cgacgggcaa taaataatgg ggagatcgtc cctttttacc aacctgtggt aaatggtcgg 600
   gaagggacat tgcggggagt tgaggtgtta gcccgctgga aacaacctca cggtggatat 660
   atatcacccg cggcatttat tccacttgct gaaaaatccg gattaatcgt tccgcttacg 720
   caaagcctga tgaatcaggt tgccagacag atgaacgcta tcgcgagtaa attgccggaa 780
   ggttttcata ttggaattaa ttttagcgct tcgcatatta tttcgccgac gtttgtcgac 840
   gagtgtttaa atttccgtga cagttttacc cgccgcgatt taaaccttgt tctggaagtc 900
   accgagcgtg agccattgaa tgttgatgaa agtctggttc agcggttgaa catactgcat 960
   gaaaatggtt ttgtcatcgc actggatgat ttcggtactg gctactcagg gctttcttat 1020
   cttcatgacc tgcatattga ttatatcaaa attgatcaca gtttcgttgg ccgcgtaaac 1080
   gcagacccag aatcaacccg aatttttggc gaaaatgaga cgttgatcgg cacgtaagag 1140
   gttccagctt tcaccataat gaaataagat cactaccggg cgtatttttt gagttatcga 1200
   gattttcagg agctaaggaa gctaaaatga ttgaacaaga tggattgcac gcaggttctc 1260
   cggccgcttg ggtggagagg ctattcggct atgactgggc acaacagaca atcggctgct 1320
   ctgatgccgc cgtgttccgg ctgtcagcgc aggggcgccc ggttcttttt gtcaagaccg 1380
   acctgtccgg tgccctgaat gaactccaag acgaggcagc gcggctatcg tggctggcca 1440
   cgacgggcgt tccttgcgca gctgtgctcg acgttgtcac tgaagcggga agggactggc 1500
   tgctattggg cgaagtgccg gggcaggatc tcctgtcatc tcaccttgct cctgccgaga 1560
   aagtatccat catggctgat gcaatgcggc ggctgcatac gcttgatccg gctacctgcc 1620
   cattcgacca ccaagcgaaa catcgcatcg agcgagcacg tactcggatg gaagccggtc 1680
   ttgtcgatca ggatgatctg gacgaagagc atcaggggct cgcgccagcc gaactgttcg 1740
   ccaggctcaa ggcgcggatg cccgacggcg aggatctcgt cgtgacccat ggcgatgcct 1800
   gcttgccgaa tatcatggtg gaaaatggcc gcttttctgg attcatcgac tgtggccggc 1860
   tgggtgtggc ggaccgctat caggacatag cgttggctac ccgtgatatt gctgaagagc 1920
   ttggcggcga atgggctgac cgcttcctcg tgctttacgg tatcgccgct cccgattcgc 1980
   agcgcatcgc cttctatcgc cttcttgacg agttcttctg atttttttaa ggcagttatt 2040
   ggtgcctcac tgattaagca ttggtaactg tcagatccgg ggaatttatg ggattcacct 2100
   ttatgttgat aagaaataaa agaaaatgcc aataggatat cggcattttc ttttgcgttt 2160
   ttatttgtta actgttaatt gtccttgttc aaggatgctg tctttgacaa cagatgtttt 2220
   cttgcctttg atgttcagca ggaagctcgg cgcaaacgtt gattgtttgt ctgcgtagaa 2280
   tcctctgttt gtcatatagc ttgtaatcac gacattgttt cctttcgctt gaggtacagc 2340
   gaagtgtgag taagtaaagg ttacatcgtt aggatcaaga tccattttta acacaaggcc 2400
   agttttgttc agcggcttgt atgggccagt taaagaatta gaaacataac caagcatgta 2460
   aatatcgtta gacgtaatgc cgtcaatcgt catttttgat ccgcgggagt cagtgaacag    2520
   ataccatttg ccgttcattt taaagacgtt cgcgcgttca atttcatctg ttactgtgtt    2580
   agatgcaatc agcggtttca tcactttttt cagtgtgtaa tcatcgttta gctcaatcat 2640
   accgagagcg ccgtttgcta actcagccgt gcgtttttta tcgctttgca gaagtttttg 2700
   actttcttga cggaagaatg atgtgctttt gccatagtat gctttgttaa ataaagattc 2760
   ttcgccttgg tagccatctt cagttccagt gtttgcttca aatactaagt atttgtggcc 2820
   tttatcttct acgtagtgag gatctctcag cgtatggttg tcgcctgagc tgtagttgcc 2880
   ttcatcgatg aactgctgta cattttgata cgtttttccg tcaccgtcaa agattgattt 2940
   ataatcctct acaccgttga tgttcaaaga gctgtctgat gctgatacgt taacttgtgc 3000
   agttgtcagt gtttgtttgc cgtaatgttt accggagaaa tcagtgtaga ataaacggat 3060
   ttttccgtca gatgtaaatg tggctgaacc tgaccattct tgtgtttggt cttttaggat 3120
   agaatcattt gcatcgaatt tgtcgctgtc tttaaagacg cggccagcgt ttttccagct 3180
   gtcaatagaa gtttcgccga ctttttgata gaacatgtaa atcgatgtgt catccgcatt 3240
   tttaggatct ccggctaatg caaagacgat gtggtagccg tgatagtttg cgacagtgcc 3300
   gtcagcgttt tgtaatggcc agctgtccca aacgtccagg ccttttgcag aagagatatt 3360
   tttaattgtg gacgaatcaa attcaggaac ttgatatttt tcattttttt gctgttcagg 3420
   gatttgcagc atatcatggc gtgtaatatg ggaaatgccg tatgtttcct tatatggctt 3480
   ttggttcgtt tctttcgcaa acgcttgagt tgcgcctcct gccagcagtg cggtagtaaa 3540
   ggttaatact gttgcttgtt ttgcaaactt tttgatgttc atcgttcatg tctccttttt 3600
   tatgtactgt gttagcggtc tgcttcttcc agccctcctg tttgaagatg gcaagttagt 3660
   tacgcacaat aaaaaaagac ctaaaatatg taaggggtga cgccaaagta tacactttgc 3720
   cctttacaca ttttaggtct tgcctgcttt atcagtaaca aacccgcgcg atttactttt 3780
   cgacctcatt ctattagact ctcgtttgga ttgcaactgg tctattttcc tcttttgttt 3840
   gatagaaaat cataaaagga tttgcagact acgggcctaa agaactaaaa aatctatctg 3900
   tttcttttca ttctctgtat tttttatagt ttctgttgca tgggcataaa gttgcctttt 3960
   taatcacaat tcagaaaata tcataatatc tcatttcact aaataatagt gaacggcagg 4020
   tatatgtgat gggttaaaaa ggatccgcta gggataacag ggtaatatag atcctctaga 4080
   gtcgacctgc aggcatgcaa gcttggcact ggctgatcag ctagcccatg ggtatgatcc 4140
   agctctggta ttccgcaaaa gcagagtacc tggcgggagc gagatatgcc gccaacaata 4200
   tcaatcatat acttgaagaa gcgtcacaag cgactcaaac agcggttaac attgccggga 4260
   aggaatgcaa cctcgaggag caatatcagc ttggcactga agcagctctg aaacctcacc 4320
   tgcgcacaat catcattctc aaacagggaa tagtctggtg tacatccctg cctgggaatc 4380
   gggtcctgtt gtctcgtatt cctgttttcc cggacagtaa tttactgttg gctccagcaa 4440
   tcgacaccgt taatagatta cctatcctgc tctatcagaa ccaatttgca gatacgcgca 4500
   ttttggttac gataagcgat cagcatattc gtggggcact taatgtaccc ttgaaagggg 4560
   taaggtatgt attacgcgtg gcggatgaca ttattgg 4597
<210> 57
   <211> 6549
   <212> DNA
   <213> Artificial Sequence
<220>
   <223> b1867-b1901
<400> 57
   tgcgctggtg gtgatcgatt tacaagaagg catcttacct tttgccggtg gtccacatac     60
   tgccgatgag gtagttaatc gcgccgggaa gctggcggcg aaatttcgcg ccagcggtca    120
   gcccgtgttt ctggtgcgcg ttggctggtc tgccgattac gctgaagcat taaaacagcc    180
   ggtcgatgcg ccctcaccgg ctaaagtgtt gcccgaaaat tggtggcaac atcctgctgc    240
   attaggtgca accgacagcg atatcgaaat catcaaacgt caatggggtg cgttttacgg    300
   tacggatctg gagttgcaat tacgccgccg aggtatcgat acaatagtgt tatgtgggat    360
   ctcgaccaat atcggtgttg aatccaccgc ccgcaatgcc tgggaacttg gctttaatct    420
   ggtgattgcc gaagacgcct gtagtgccgc cagcgccgag cagcacaata acagtatcaa    480
   ccatatctac ccgcgcatcg cccgtgtgcg tagcgtggaa gagatcctca acgcgtttac    540
   cctgttgccc tgtgccaacc aaccgctgat ttcacgccgc ttctgatgca atagtgaaaa    600
   cggcaatacg ccacgcgcac gttgctgacg aaaacagcca tttgcagtat actcccgccc    660
   taatttcttt aactggtgcg ggcaattttt gctcgcttca tcaatgtaag gtattccggt    720
   gaatattcag gctcttctct cagaaaaagt ccgtcaggcc atgattgcgg caggcgcgcc    780
   tgcggattgc gaaccgcagg ttcgtcagtc agcaaaagtt cagttcggcg actatcaggc    840
   taacggcatg atggcagttg ctaaaaaact gggtatggca ccgcgacaat tagcagagca    900
   ggtgctgact catctggatc ttaacggtat cgccagcaaa gttgagatcg ccggtccagg    960
   ctttatcaac attttccttg atccggcatt cctggctgaa catgttcagc aggcgctggc   1020
   gtccgatcgt ctcggtgttg ctacgccaga aaaacagacc attgtggttg actactctgc   1080
   gccaaacgtg gcgaaagaga tgcatgtcgg tcacctgcgc tctaccatta ttggtgacgc   1140
   agcagtgcgt actctggagt tcctcggtca caaagtgatt cgcgcaaacc acgtcggcga   1200
   ctggggcact cagttcggta tgctgattgc atggctggaa aagcagcagc aggaaaacgc   1260
   cggtgaaatg gagctggctg accttgaagg tttctaccgc gatgcgaaaa agcattacga   1320
   tgaagatgaa gagttcgccg agcgcgcacg taactacgtg gtaaaactgc aaagcggtga   1380
   cgaatatttc cgcgagatgt ggcgcaaact ggtcgacatc accatgacgc agaaccagat   1440
   cacctacgat cgtctcaacg tgacgctgac ccgtgatgac gtgatgggcg aaagcctcta   1500
   caacccgatg ctgccaggaa ttgtggcgga tctcaaagcc aaaggtctgg cagtagaaag 1560
   cgaaggggcg accgtcgtat tccttgatga gtttaaaaac aaggaaggcg aaccgatggg 1620
   cgtgatcatt cagaagaaag atggcggcta tctctacacc accactgata tcgcctgtgc 1680
   gaaatatcgt tatgaaacac tgcatgccga tcgcgtgctg tattacatcg actcccgtca 1740
   gcatcaacac ctgatgcagg catgggcgat cgtccgtaaa gcaggctatg taccggaatc 1800
   cgtaccgctg gaacaccaca tgttcggcat gatgctgggt aaagacggca aaccgttcaa 1860
   aacccgcgcg ggtggtacag tgaaactggc cgatctgctg gatgaagccc tggaacgtgc 1920
   acgccgtctg gtggcagaaa agaacccgga tatgccagcc gacgagctgg aaaaactggc 1980
   taacgcggtt ggtattggtg cggtgaaata tgcggatctc tccaaaaacc gcaccacgga 2040
   ctacatcttc gactgggaca acatgctggc gtttgagggt aataccgcgc catacatgca 2100
   gtatgcatac acgcgtgtat tgtccgtgtt ccgtaaagca gaaattgacg aagagcaact 2160
   ggctgcagct ccggttatca tccgtgaaga tcgtgaagcg caactggcag ctcgcctgct 2220
   gcagtttgaa gaaaccctca ccgtggttgc ccgtgaaggc acgccgcatg taatgtgtgc 2280
   ttacctgtac gatctggccg gtctgttctc tggcttctac gagcactgcc cgatcctcag 2340
   cgcagaaaac gaagaagtgc gtaacagccg tctaaaactg gcacaactga cggcgaagac 2400
   gctgaagctg ggtctggata cgctgggtat tgagactgta gagcgtatgt aatcgatttt 2460
   tcgtgagagt gaagcctgat cagggttagc cgatctccat aggagagcaa tatcacatcg 2520
   cagaattaca gtgagaacgt gcataaattt agcgggaaaa gacataaggg aaagccaatt 2580
   tgtcagacaa attgtcgaat gcacagcaga ttaatccata agattagcct ggaaatcctt 2640
   gttgtctttg gtacccatgc gggatgtctt cttttaacca agtcaatagg ccgcattacc 2700
   tggcgttgag tttttgaaat ggtgtaataa ccgcaactca aagatgtgga aaatgcacgt 2760
   cattcatttc gtcattaatt atcactgtgc tcattaatta acagaacacg tataatgaga 2820
   gccatctcgc aaaaatgaaa aaacgtttta taaaatcatc acttcatcat gaattcaaat 2880
   tcattgatta atatcaacaa gatacaaaaa gcactatcat taaaattcat tgcagttaca 2940
   ttgatttcat caatgaaatg taaaaatata taaacttgat gatttaagca ttttcttata 3000
   cccgttcaga cgttattctt atttcagatc atcgtcagaa ttgactccac gatcacattt 3060
   ttggcgaaaa tgagacgttg atcggcacgt aagaggttcc agctttcacc ataatgaaat 3120
   aagatcacta ccgggcgtat tttttgagtt atcgagattt tcaggagcta aggaagctaa 3180
   aatggagaaa aaaatcactg gatataccac cgttgatata tcccaatggc atcgtaaaga 3240
   acattttgag gcatttcagt cagttgctca atgtacctat aaccagaccg ttcagctgga 3300
   tattacggcc tttttaaaga ccgtaaagaa aaataagcac aagttttatc cggcctttat 3360
   tcacattctt gcccgcctga tgaatgctca tccgaaattc cgtatggcaa tgaaagacgg 3420
   tgagctggtg atatgggata gtgttcaccc ttgttacacc gttttccatg agcaaactga 3480
   aacgttttca tcgctctgga gtgaatacca cgacgatttc cggcagtttc tacacatata 3540
   ttcgcaagat gtggcgtgtt acggtgaaaa cctggcctat ttccctaaag ggtttattga 3600
   gaatatgttt ttcgtctcag ccaatccctg ggtgagtttc accagttttg atttaaacgt 3660
   ggccaatatg gacaacttct tcgcccccgt tttcaccatg ggcaaatatt atacgcaagg 3720
   cgacaaggtg ctgatgccgc tggcgattca ggttcatcat gccgtctgtg atggcttcca 3780
   tgtcggcaga atgcttaatg aattacaaca gtactgcgat gagtggcagg gcggggcgta 3840
   atttttttaa ggcagttatt ggtgcctcac tgattaagca ttggtaactg tcagatccgg 3900
   ggaatttatg ggattcacct ttatgttgat aagaaataaa agaaaatgcc aataggatat 3960
   cggcattttc ttttgcgttt ttatttgtta actgttaatt gtccttgttc aaggatgctg 4020
   tctttgacaa cagatgtttt cttgcctttg atgttcagca ggaagctcgg cgcaaacgtt 4080
   gattgtttgt ctgcgtagaa tcctctgttt gtcatatagc ttgtaatcac gacattgttt 4140
   cctttcgctt gaggtacagc gaagtgtgag taagtaaagg ttacatcgtt aggatcaaga 4200
   tccattttta acacaaggcc agttttgttc agcggcttgt atgggccagt taaagaatta 4260
   gaaacataac caagcatgta aatatcgtta gacgtaatgc cgtcaatcgt catttttgat 4320
   ccgcgggagt cagtgaacag ataccatttg ccgttcattt taaagacgtt cgcgcgttca 4380
   atttcatctg ttactgtgtt agatgcaatc agcggtttca tcactttttt cagtgtgtaa 4440
   tcatcgttta gctcaatcat accgagagcg ccgtttgcta actcagccgt gcgtttttta 4500
   tcgctttgca gaagtttttg actttcttga cggaagaatg atgtgctttt gccatagtat 4560
   gctttgttaa ataaagattc ttcgccttgg tagccatctt cagttccagt gtttgcttca 4620
   aatactaagt atttgtggcc tttatcttct acgtagtgag gatctctcag cgtatggttg 4680
   tcgcctgagc tgtagttgcc ttcatcgatg aactgctgta cattttgata cgtttttccg 4740
   tcaccgtcaa agattgattt ataatcctct acaccgttga tgttcaaaga gctgtctgat 4800
   gctgatacgt taacttgtgc agttgtcagt gtttgtttgc cgtaatgttt accggagaaa 4860
   tcagtgtaga ataaacggat ttttccgtca gatgtaaatg tggctgaacc tgaccattct 4920
   tgtgtttggt cttttaggat agaatcattt gcatcgaatt tgtcgctgtc tttaaagacg 4980
   cggccagcgt ttttccagct gtcaatagaa gtttcgccga ctttttgata gaacatgtaa 5040
   atcgatgtgt catccgcatt tttaggatct ccggctaatg caaagacgat gtggtagccg 5100
   tgatagtttg cgacagtgcc gtcagcgttt tgtaatggcc agctgtccca aacgtccagg 5160
   ccttttgcag aagagatatt tttaattgtg gacgaatcaa attcaggaac ttgatatttt 5220
   tcattttttt gctgttcagg gatttgcagc atatcatggc gtgtaatatg ggaaatgccg 5280
   tatgtttcct tatatggctt ttggttcgtt tctttcgcaa acgcttgagt tgcgcctcct 5340
   gccagcagtg cggtagtaaa ggttaatact gttgcttgtt ttgcaaactt tttgatgttc 5400
   atcgttcatg tctccttttt tatgtactgt gttagcggtc tgcttcttcc agccctcctg 5460
   tttgaagatg gcaagttagt tacgcacaat aaaaaaagac ctaaaatatg taaggggtga 5520
   cgccaaagta tacactttgc cctttacaca ttttaggtct tgcctgcttt atcagtaaca 5580
   aacccgcgcg atttactttt cgacctcatt ctattagact ctcgtttgga ttgcaactgg 5640
   tctattttcc tcttttgttt gatagaaaat cataaaagga tttgcagact acgggcctaa 5700
   agaactaaaa aatctatctg tttcttttca ttctctgtat tttttatagt ttctgttgca 5760
   tgggcataaa gttgcctttt taatcacaat tcagaaaata tcataatatc tcatttcact 5820
   aaataatagt gaacggcagg tatatgtgat gggttaaaaa ggatccgcta gggataacag 5880
   ggtaatatag atcctctaga gtcgacctgc aggcatgcaa gcttggcact ggctgatcag 5940
   ctagcccatg ggtatgacca gcattgagtt ggcagcgtca aatgcccccg ggatgtcgtt 6000
   agatttggta ggtacctgat aaatttgttt ttccgggaat ccggccgctt tcagcgcatc    6060
   catagatccc gtagtacggc ggcgggcggt atccagttcg ttggcggtaa tcgccatcac    6120
   cgcgctttct ttgacatccc agccacgttt ctgcatctct ttatacagtt cctggccctg    6180
   acgttcgcca attttagtcg ccgccatcat caccagcgga acggtatcca ttggcttacc    6240
   tttggcgtta acaaactggt catccacggc aatgactttc atatcgtagc cacgcgcttt    6300
   cgcgacgatg gcagagccga gtttggggtc cggagtacaa ataacgaaac cttttgcgcc    6360
   actggcagcc aggctgtcga tcgcgttcaa tgttttttcg ccatccggca cggcaatctt     6420
   aataacctca aaccctaaat ccttcccggc tttatcggca aacttccatt cggtctggaa    6480
   ccacggctct tccggttgct tcaccagaaa accgagcttc aggttctccg ccatagcgga    6540
   ttgtgacat                                                           6549